# EUROPEAN PATENT SPECIFICATION

(11) **EP 1 804 732 B1**
(45) Date of publication and mention of the grant of the patent: **14.09.2011**
(21) Application number: 05798791.9
(22) Date of filing: 23.09.2005
(51) Int. Cl.: A61F 2/44, A61B 17/70

(54) **INTERSPINOUS PROCESS IMPLANT INCLUDING A BINDER**
PROCESSUS INTERSPINOSUS-IMPLANTAT MIT EINEM BINDEMITTEL
IMPLANT ENTRE APOPHYSES EPINEUSES COMPORTANT UN LIEN

(30) Priority: 23.09.2004 US 612465 P; 31.03.2005 US 95440; 31.03.2005 US 95680
(43) Date of publication of application: 11.07.2007
(73) Proprietor: Kyphon SÀRL, 2000 Neuchâtel (CH)
(72) Inventor: ZUCHERMAN, James, F., San Francisco, California 94118 (US); HSU, Ken, Y., San Francisco, California 94114 (US); KLYCE, Henry, A., Piedmont, California 94611 (US); WINSLOW, Charles, J., Walnut Creek, California 94595 (US); FLYNN, John, J., West Milford, New Jersey 07480 (US); MITCHELL, Steven, T., Pleasant Hill, California 94523 (US); YERBY, Scott, A., Montara, California 94037 (US); MARKWART, John, A., Castro Valley, California 94552 (US)
(74) Representative: Price, Nigel John King
(86) International application number: PCT/US2005/034057
(87) International publication number: WO 2006/034423

(56) References cited:
- WO-A1-02/051326
- US-A- 5 496 318
- US-A- 5 645 599
- US-A1- 2002 040 223
- US-B2- 6 582 433

## Description

### TECHNICAL FIELD

This invention relates to interspinous process implants.

### BACKGROUND OF THE INVENTION

As the present society ages, it is anticipated that there will be an increase in adverse spinal conditions which are characteristic of older people. Certain biochemical changes can occur with aging, affecting tissue found throughout the body. In the spine, the structure of the intervertebral disks can be compromised, in part as the structure of the annulus fibrosus of the intervertebral disk weakens due to degenerative effects. Spondylosis (also referred to as spinal osteoarthritis) is one example of a degenerative disorder that can cause loss of normal spinal structure and function. The degenerative process can impact the cervical, thoracic, and/or lumbar regions of the spine, affecting the intervertebral disks and the facet joints. Pain associated with degenerative disorders is often triggered by one or both of forward flexion and hyperextension. Spondylosis in the thoracic region of the spine can cause disk pain during flexion and facet pain during hyperextension. Spondylosis can affect the lumbar region of the spine, which carries most of the body's weight, and movement can stimulate pain fibers in the annulus fibrosus and facet joints.

Over time, loss of disk height can result in a degenerative cascade with deterioration of all components of the motion segment resulting in segment instability and ultimately in spinal stenosis (including, but not limited to, central canal and lateral stenosis). Spinal stenosis results in a reduction in foraminal area (*i.e.*, the available space for the passage of nerves and blood vessels) which compresses the nerve roots and causes radicular pain. Another symptom of spinal stenosis is myelopathy. Extension and ipsilateral rotation further reduces the foraminal area and contributes to pain, nerve root compression and neural injury. During the process of deterioration, disks can become herniated and/or become internally torn and chronically painful. When symptoms seem to emanate from both anterior (disk) and posterior (facets and foramen) structures, patients cannot tolerate positions of extension or flexion.

A common procedure for handling pain associated with degenerative spinal disk disease is the use of devices for fusing together two or more adjacent vertebral bodies. The procedure is known by a number of terms, one of which is interbody fusion. Interbody fusion can be accomplished through the use of a number of devices and methods known in the art. These include screw arrangements, solid bone implant methodologies, and fusion devices which include a cage or other mechanism which is packed with bone and/or bone growth inducing substances. All of the above are implanted between adjacent vertebral bodies in order to fuse the vertebral bodies together, alleviating associated pain.

Depending on the degree of slip and other factors, a physician may fuse the vertebra "as is," or fuse the vertebrae and also use a supplemental device. Supplemental devices are often associated with primary fusion devices and methods, and assist in the fusion process. Supplemental devices assist during the several month period when bone from the adjacent vertebral bodies is growing together through the primary fusion device in order to fuse the adjacent vertebral bodies. During this period it is advantageous to have the vertebral bodies held immobile with respect to each other so that sufficient bone growth can be established. Supplemental devices can include hook and rod arrangements, screw arrangements, and a number of other devices which include straps, wires, and bands, all of which are used to immobilize one portion of the spine relative to another. Supplemental devices have the disadvantage that they generally require extensive surgical procedures in addition to the extensive procedure surrounding the primary fusion implant. Such extensive surgical procedures include additional risks, including risk of causing damage to the spinal nerves during implantation. Spinal fusion can include highly invasive surgery requiring use of a general anesthetic, which itself includes additional risks. Risks further include the possibility of infection, and extensive trauma and damage to the bone of the vertebrae caused either by anchoring of the primary fusion device or the supplemental device. Finally, spinal fusion can result in an absolute loss of relative movement between vertebral bodies.

U.S. Pat. No. 5,496,318 to Howland, et al. teaches supplemental devices for the stabilization of the spine for use with surgical procedures to implant a primary fusion device. *Howland* '318 teaches an H-shaped spacer having two pieces held together by a belt, steel cable, or polytetrafluoroethane web material, one or both ends of which includes an attachment device fixedly connected with the respective end. *Howland* '318 teaches that the vertebra are preferably surgically modified to include a square notch to locate the fixation device in a preferred location. *Howland* '318 has the further disadvantage that the belt, cable or web material must be sized before implantation, increasing the procedure time to include sizing time and reducing the precision of the fit where both ends of the belt, cable or web material include attachment devices (and as such are incrementally sized).

U.S. Pat. No. 5,609,634 to Voydeville teaches a prosthesis including a semi-flexible interspinous block positioned between adjacent spinous processes and a ligament made from the same material. A physician must lace the ligament through the interspinous block and around the spinous processes in a figure of eight, through the interspinous block and around the spinous processes in an oval, and suture the ligament to itself to fix the interspinous block in place. *Voydeville* has the disadvantage of requiring significant displacement and/or removal of tissue associated with the spinous processes, potentially resulting in significant trauma and damage. *Voydeville* has the further disadvantage of requiring the physician to lace the interspinous ligament through the interspinous block. Such a procedure can require care and time, particularly because a physician's ability to view the area of interest is complicated by suffusion of blood in the area of interest.

It would be advantageous if a device and procedure for limiting flexion and extension of adjacent vertebral bodies were as simple and easy to perform as possible, and would preferably (though not necessarily) leave intact all bone, ligament, and other tissue which comprise and surround the spine. Accordingly, there is a need for procedures and implants which are minimally invasive and which can supplement or substitute for primary fusion devices and methods, or other spine fixation devices and methods. Accordingly, a need exists to develop spine implants that alleviate pain caused by spinal stenosis and other such conditions caused by damage to, or degeneration of, the spine. Such implants would distract (increase) or maintain the space between the vertebrae to increase the foraminal area and reduce pressure on the nerves and blood vessels of the spine, and limit or block flexion to reduce pain resulting from spondylosis and other such degenerative conditions.

A further need exists for development of a minimally invasive surgical implantation method for spine implants that preserves the physiology of the spine. A still further need exists for an implant that accommodates the distinct anatomical structures of the spine, minimizes further trauma to the spine, and obviates the need for invasive methods of surgical implantation. Additionally, a need exists to address adverse spinal conditions that are exacerbated by spinal extension and flexion.

U.S. Pat. No. 5 496 318 discloses a spinal fixation device comprising an H-shaped spacer for insertion between adjacent spinous processes. The spacer comprises a first element forming one side of the H-shape and a second element forming another side of the H shape. The first element interlocks with the second element at the crossbar of the H-shape. A belt encircles adjacent spinous processes and the installed spacer. A means is provided for attaching the belt to the outside of the spacer.

WO-A-02/051326 discloses an intervertebral implant comprising a wedge designed to be pressed between two spinous processes. The wedge has two opposite grooves each defined by two wings, the axes of the two grooves being substantially mutually parallel and the spinous processes being urged to be supported in said two grooves. The wedge comprises at least a central recess between said two grooves, this central recess passing right through said wedge along an axis Ac substantially parallel to the axes Ag1 and Ag2 of said grooves, thereby making said wedge elastically deformable.

The present invention relates to an implant as claimed hereafter. Preferred embodiments of the invention are set forth in the dependent claims.

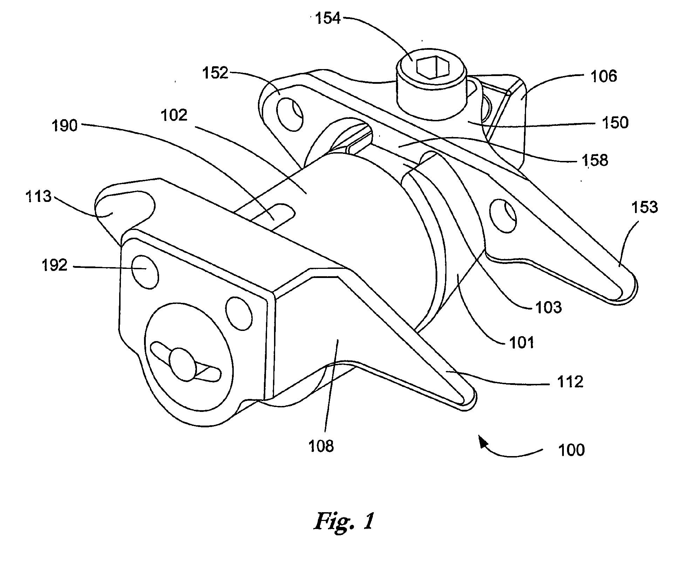

Embodiments of apparatus in accordance with the present invention will now be described, by way of example only, with reference to the accompanying drawings. The implants illustrated in Figs. 1-2C do not form part of the present invention, but will present background art that is useful for understanding the invention. In the drawings:
**FIG. 1** is a perspective view of an interspinous implant capable of limiting or blocking relative movement of adjacent spinous processes during extension of the spine.
**FIG. 2A** is a posterior view of the implant of **FIG. 1** positioned between adjacent spinous processes.
**FIG. 2B** is a cross-sectional side view of a spacer of the interspinous implant of **FIGs. 1** and **2A** positioned between spinous processes.
**FIG. 2C** is a cross-sectional view of the spacer of **FIG. 2B** during flexion of the spine.
**FIG. 3A** is a side view of an embodiment of an implant in accordance with the present invention having a distraction guide, a spacer, a brace, and a binder associated with the brace and fixable in position by a capture device.
**FIG. 3B** is a side view of an alternative embodiment of an implant in accordance with the present invention including a brace wall having recesses for receiving lobes of a capture device.
**FIG. 3C** is a side view of still another embodiment of an implant in accordance with the present invention including a capture device having a spring-loaded can for securing a binder against a brace wall.
**FIG. 3D** is a side view of a still further embodiment of an implant in accordance with the present invention including a capture device having dual spring-loaded cams for securing a binder in position.
**FIG. 4A** is an end view of the implant of **FIG. 3A** positioned between adjacent spinous processes.
**FIG. 4B** is an end view of the implant of **FIG. 3A** positioned between adjacent spinous processes.
**FIG. 4C** is an end view of the implant of **FIG. 3A** positioned between adj acent spinous processes wherein the spinous processes are surgically modified to receive a binder.
**FIG. 5** is an end view of an alternative embodiment of an implant in accordance with the present invention having a binder that varies in shape along the binder's length.
**FIG. 6A** is an end view of the implant of **FIG. 5** positioned between adjacent spinous processes.
**FIG. 6B** is an opposite end view of the implant of **FIG. 6A****.**
**FIG. 6C** is an end view of still another embodiment of an implant in accordance with the present invention having a cord for a binder.
**FIG. 7A** is a side view of an embodiment of an implant in accordance with the present invention including a wing associated with the distraction guide to further limit or block movement of the implant.
**FIG. 7B** is a partial cross-sectional side view of an alternative embodiment of an implant in accordance with the present invention include an extendable wing associated with the distraction guide, the extendable wing being in a retracted position.
**FIG. 7C** is a partial cross-sectional side view of the implant of **FIG. 7B** wherein the extendable wing is in an extended position.
**FIG. 7D** is a partial cross-sectional side view of still another embodiment of an implant in accordance with the present invention including a spring-loaded wing associated with the distraction guide, the wing being in an extended position.
**FIG. 7E** is a partial cross-sectional side view of the implant of **FIG. 7D** wherein the spring-loaded wing is in a collapsed position.
**FIG. 8** is a top view of two implants in accordance with an embodiment of the present invention positioned between the spinous processes of adjacent vertebrae, one of the implants having a binder arranged around the adjacent spinous processes.
**FIG. 9A** is a perspective view of a further embodiment of an implant in accordance with the present invention having a distraction guide, a spacer, a brace, and a binder associated with the brace and fixable in position by a capture device.
**FIG. 9B** is a perspective view the implant of **FIG. 9A** wherein the capture device is arranged to secure a binder between the capture device and the brace.
**FIG. 9C** is a side view of the implant of **FIGs. 9A** and **9B****.**
**FIG. 10A** is a cross-sectional top view of a binder loosely positioned within the capture device of the implant of **FIGs. 9A** and **9B****.**
**FIG. 10B** is a cross-sectional top view of the binder secured to the brace by the capture device of the implant of **FIGs. 9A** and **9B****.**
**FIG. 10C** is a cross-sectional top view of a binder loosely positioned within an alternative embodiment of a capture device of the implant of **FIGs. 9A** and **9B****.**
**FIG. 10D** is a cross-sectional top view of the binder and capture device of **FIG. 10C** wherein the binder is secured to the brace.
**FIG.11** is an end view of the implant of **FIGs. 9A** and **9B** positioned between adjacent spinous processes.
**FIG.12** is a block diagram illustrating a method of positioning the implant of **FIGs. 9A-11** between adjacent spinous processes.
**FIG. 13A** is a perspective view of an still another embodiment of an implant in accordance with the present invention having a distraction guide, a spacer, a first wing, and a second wing including a capture device.
**FIG. 13B** is a perspective view of the implant of **FIG. 13A** in accordance with the present invention having a distraction guide, a spacer, a first wing, and a second wing including a capture device.
**FIG. 14** is a perspective view of an still another embodiment of an implant in accordance with the present invention having a distraction guide, a spacer, a first wing, and a second wing including a capture device.
**FIG. 15** is a perspective view of an still another embodiment of an implant in accordance with the present invention having a distraction guide, a spacer, a first wing, and a second wing including a capture device.
**FIG.16** is a block diagram illustrating a method of positioning the implant of **FIGs.** 13A-15 between adjacent spinous processes.

### DETAILED DESCRIPTION

**FIG. 1** is a perspective view of an implant as described in U.S. Pat. 6,695,842 to Zucherman, et al. and U.S. Pat. 6,712,819 to Zucherman et al. The implant **100** has a main body 101. The main body **101** includes a spacer **102,** a first wing **108,** a lead-in tissue expander **106** (also referred to herein as a distraction guide) and an alignment track **103.** The main body **101** is inserted between adjacent spinous processes. Preferably, the main body **101** remains (where desired) in place without attachment to the bone or ligaments.

The distraction guide **106** includes a tip from which the distraction guide **106** expands, the tip having a diameter sufficiently small such that the tip can pierce an opening in an interspinous ligament and/or can be inserted into a small initial dilated opening. The diameter and/or cross-sectional area of the distraction guide **106** then gradually increases until it is substantially similar to the diameter of the main body **101** and spacer **102.** The tapered front end eases the ability of a physician to urge the implant **100** between adjacent spinous processes. When urging the main body **101** between adjacent spinous processes, the front end of the distraction guide **106** distracts the adjacent spinous processes and dilates the interspinous ligament so that a space between the adjacent spinous processes is approximately the diameter of the spacer **102.**

The shape of the spacer **102** is such that for purposes of insertion between the spinous processes, the spinous processes need not be altered or cut away in order to accommodate the spacer **102.** Additionally, associated ligaments need not be cut away and there is little or no damage to the adjacent or surrounding tissues. As shown in **FIG. 1****,** the spacer **102** is elliptically shaped in cross-section, and can swivel about a central body (also referred to herein as a shaft) extending from the first wing **108** so that the spacer **102** can self-align relative to the uneven surfaces of the spinous processes. Self-alignment can ensure that compressive loads are distributed across the surface of the bone. As contemplated in Zucherman '842, the spacer **102** can have, for example, a diameter of six millimeters, eight millimeters, ten millimeters, twelve millimeters and fourteen millimeters. These diameters refer to the height by which the spacer distracts and maintains apart the spinous process. For an elliptically shaped spacer, the selected height (i.e., diameter) is the minor dimension measurement across the ellipse. The major dimension is transverse to the alignment of the spinous process, one above the other.

The first wing **108** has a lower portion **113** and an upper portion **112.** As shown in **FIG. 1****,** the upper portion **112** is shaped to accommodate the anatomical form or contour of spinous processes (and/or laminae) of the L4 (for an L4-L5 placement) or L5 (for an L5-S1 placement) vertebra. The same shape or variations of this shape can be used to accommodate other motion segments. The lower portion **113** can also be rounded to accommodate the spinous processes. The lower portion **113** and upper portion **112** of the first wing **108** act as a stop mechanism when the implant **100** is inserted between adjacent spinous processes. The implant **100** cannot be inserted beyond the surfaces of the first wing **108.** Additionally, once the implant **100** is inserted, the first wing **108** can prevent side-to-side, or posterior-to-anterior movement of the implant **100.** The first wing **108** can further include one or more alignment holes **103** and one or more locking pin holes **104** for receiving pins of a main body insertion instrument (not shown).

The implant **100** further includes an adjustable wing **150** (also referred to herein as a second wing). The adjustable wing **150** has a lower portion **152** and an upper portion **153.** Similar to the first wing **108,** the adjustable wing **150** is designed to accommodate the anatomical form or contour of the spinous processes and/or lamina. The adjustable wing **150** is secured to the main body **101** with a fastener **154.** The adjustable wing **150** also has an alignment tab **158.** When the adjustable wing **150 is** initially placed on the main body **101,** the alignment tab **158** engages the alignment track **103.** The alignment tab **158** slides within the alignment track **103** and helps to maintain the adjustable wing **150** substantially parallel with the first wing **108.** When the main body **101** is inserted into the patient and the adjustable wing **150** has been attached, the adjustable wing **150** also can prevent side-to-side, or posterior-to-anterior movement.

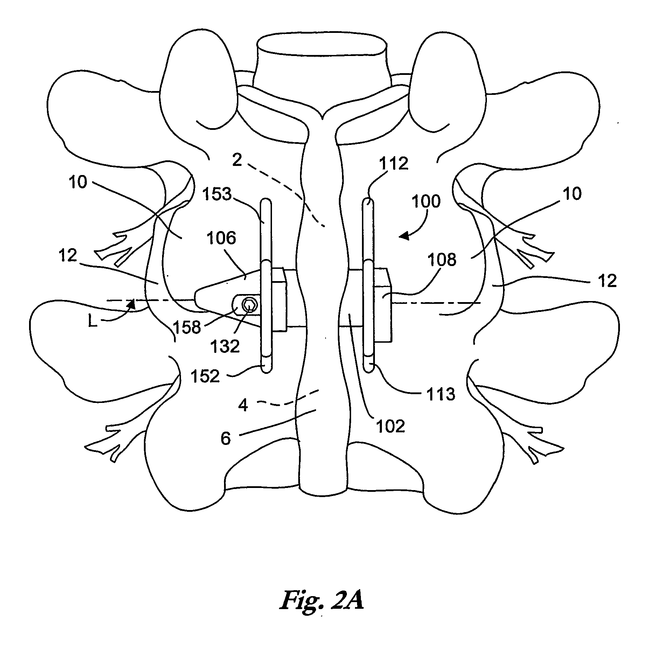

**FIG. 2A** illustrates an implant **100** positioned between adjacent spinous processes extending from vertebrae of the lumbar region. The implant **100** is positioned between inferior articular processes **10** associated with the upper vertebrae and superior articular processes **12** associated with the lower vertebrae. The superspinous ligament **6** connects the upper and lower spinous processes **2,4.** The implant **100** can be positioned without severing or otherwise destructively disturbing the superspinous ligament **6.**

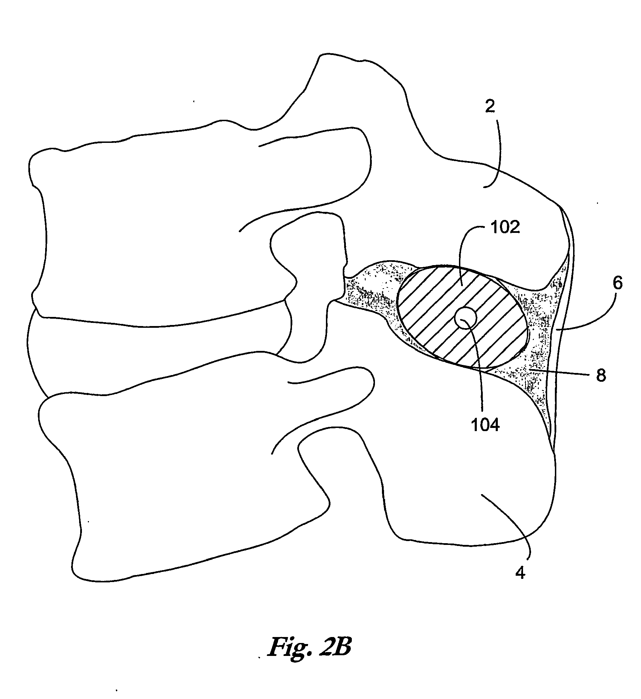

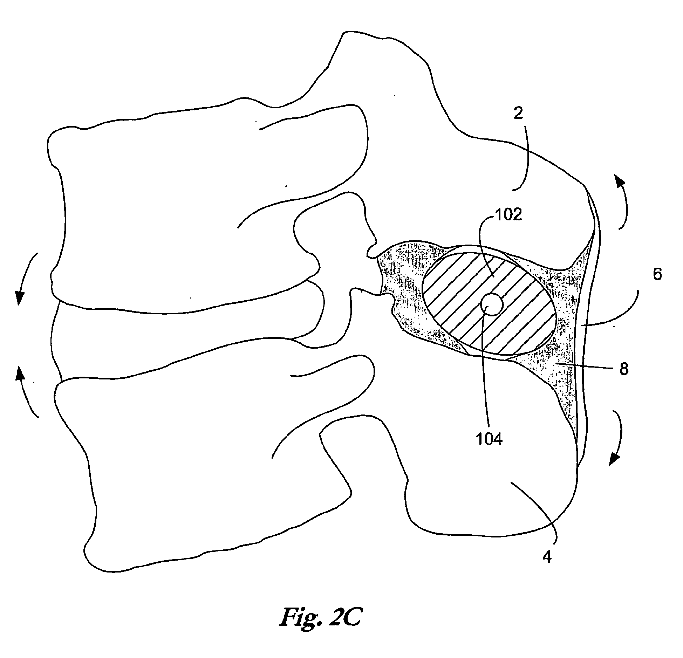

Referring to **FIG. 2B****,** the spacer **102** of the implant **100** of **FIG. 2A** is shown in cross-section. The spacer **102** defines a minimum space between adjacent spinous processes **2,4.** During extension the spacer **102** limits or blocks relative movement between the adjacent spinous processes 2,4, limiting or blocking the collapse of the space between the spinous processes 2,4. Such support can alleviate symptoms of degenerative disorder by preventing a reduction of the foraminal area and compression of the nerve roots, or by avoiding aggravation of a herniated disk, or by relieving other problems. However, as shown in **FIG. 2C****,** the implant **100** permits flexion, which in some degenerative disorders (for example in cases of spinal stenosis) can relieve some symptoms. As can be seen, during flexion the spacer **102** can float between the spinous processes, held in position by the interspinous ligament 8, and/or other tissues and structures associated with the spine. The ability to float between the spinous processes 2,4 also permits varying degrees of rotation, as well as flexion. Implants as described in Zucherman '842 thus have the advantage that they permit a greater degree of movement when compared with primary and supplementary spinal fusion devices.

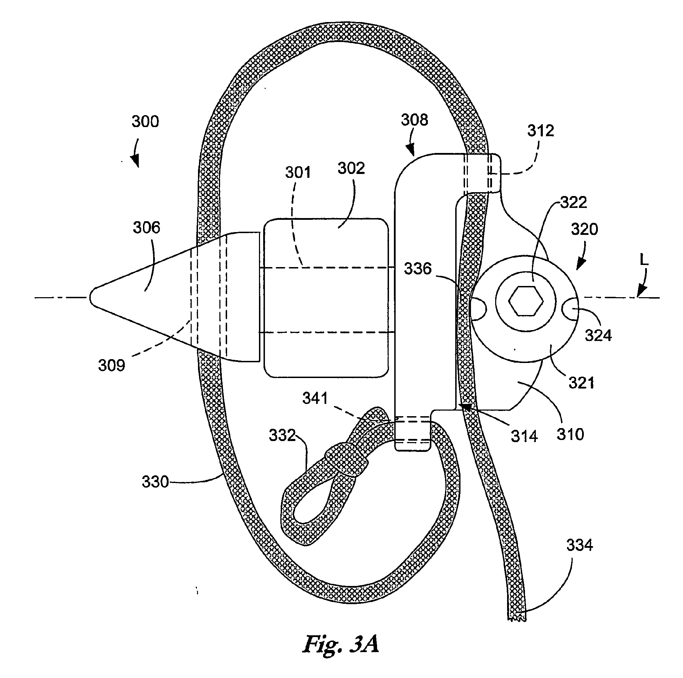

In some circumstances, for example where a patient develops spondylosis or other degenerative disorder that makes both flexion and extension painful and uncomfortable, it can be desired that the spinous processes be further immobilized, while providing the same ease of implantation as provided with implants described above. Referring to **FIG. 3A****,** an embodiment of an implant **300** in accordance with the present invention is shown. The implant **300** includes a distraction guide **306,** a spacer **302,** and a brace **308.** As shown, the spacer **302** is rotatable about a central body **301** extending from the brace **302,** although in other embodiments the spacer **302** can be fixed is position. A binder **330** can be fixedly connected with the brace **308** at a proximal end **332** of the binder **330.** The binder **330** is flexible, or semi-flexible, and can be positioned around adjacent spinous processes so that the binder **330** engages the spinous processes during flexion of the spine. Once positioned around adjacent spinous processes, tension of the binder **330** can be set when the binder **330** is secured to the brace **308** so that relative movement of the adjacent spinous processes during flexion is limited or prevented, as desired.

As can be seen in **FIG. 3A****,** in an embodiment the brace **308** can include a first end having a slot **341** through which the proximal end **332** of the binder **330** can be threaded and subsequently sutured, knotted or otherwise bound so that the proximal end **332** of the binder **330** cannot be drawn through the slot **341.** In other embodiments (not shown), the proximal end **332** can be looped or can include a connector, such as a clasp or other device, and can be fixed to the brace **308** via a fastener that engages the connector. One of ordinary skill in the art can appreciate the myriad different ways in which the proximal end **332** of the binder **330** can be associated with the brace **308** so that tension can be applied to the binder **330,** and implants in accordance with the present invention are not intended to be limited to those schemes described in detail herein.

The brace **308** can include a height along the spine greater than a height of the spacer **302** so that movement along a longitudinal axis **L** in the direction of insertion is limited or blocked by the brace **308** when the brace **308** contacts the lateral surfaces of the spinous processes. In this way, the brace **308** can function similarly to the wing **108** of the above described implant **100.** In other embodiments, the brace **308** can have a height greater or smaller than as shown. Once the binder **330** is positioned around the spinous processes and secured, movement of the implant **300** relative to the spinous processes is limited by the binder **330** along the longitudinal axis as well as along the spinous processes (i.e., anterior-to-posterior movement).

A free end of the binder **330** can be secured to the brace **308** by a capture device **320** associated with the brace **308.** The brace **308** can include a flange **310** from which the capture device **320** can extend. In the embodiment shown in **FIG. 3A****,** the capture device **320** comprises a rotatable cam **321** having a fastener **322** and one or more cut-outs **324.** A tool can be mated with the cut-outs **324** and rotated to pivot the rotatable cam **321.** When the cam **321** is rotated, the eccentric shape of the cam **321** causes a gap to close between the cam **321** and a wall **314** of the brace **330** from which the flange **310** extends. When the binder **330** is positioned between the cam **321** and the wall **314,** the rotation of the cam **321** can pinch the binder **330** between the cam **321** and the wall **314,** defining a secured end **336** of the binder **330.** Optionally, the fastener **322** can be screwed (i.e.., rotated) so that the fastener **322** is further seated, tightening against the cam **321** to fix the cam **321** in position. Further, optionally, one or both of the wall **314** and the rotatable cam **321** can include knurls, or some other texture (e.g., teeth) to prevent slippage (i.e., the slipping of the binder **330** between the cam **321** and the wall **314**). The brace **308** can further include a guide **312,** such as a channel or slot (a slot as shown) at a second end of the brace **308** to align the binder **330** with the capture device **320.**

The binder **330** can comprise a strap, ribbon, tether, cord, or some other flexible (or semi-flexible), and preferably threadable structure. The binder **330** can be made from a biocompatible material. In an embodiment, the binder **330** can be made from a braided polyester suture material. Braided polyester suture materials include, for example, Ethibond, Ethiflex, Mersilene, and Dacron, and are nonabsorbable, having high tensile strength, low tissue reactivity and improved handling. In other embodiments, the binder **330** can be made from stainless steel (i.e., surgical steel), which can be braided into a tether or woven into a strap, for example. In still other embodiments, the binder **330** can be made from some other material (or combination of materials) having similar properties.

The distraction guide **306** can optionally include a slot, bore, cut-out or other cavity **309** formed in the distraction guide **306** through which the binder **330** can be threaded or positioned. Such a cavity can allow on-axis positioning of the binder **330** (i.e., the binder can be substantially aligned with the longitudinal axis **L** of the implant **300**). Further, capturing the binder **330** within a slot or bore can prevent or limit shifting of the distraction guide 306 relative to the binder **330** to further secure the implant **300** between the spinous processes.

As will be readily apparent to one of skill in the art, implants in accordance with the present invention provide significant benefits to a physician by simplifying an implantation procedure and reducing procedure time, while providing an implant that can limit or block flexion and extension of the spine. A physician can position an implant between adjacent spinous processes and can position a binder **330** connected with the brace **308** around the spinous processes without requiring the physician to measure an appropriate length of the binder **330** prior to implantation. The capture device **320** allows the binder **330** to be secured to the brace **308** anywhere along a portion of the binder **330,** the portion being between a distal end **334** of the binder **330** and the proximal end **332.** The physician can secure the binder **330** to the brace **308** to achieve the desired range of movement (if any) of the spinous processes during flexion.

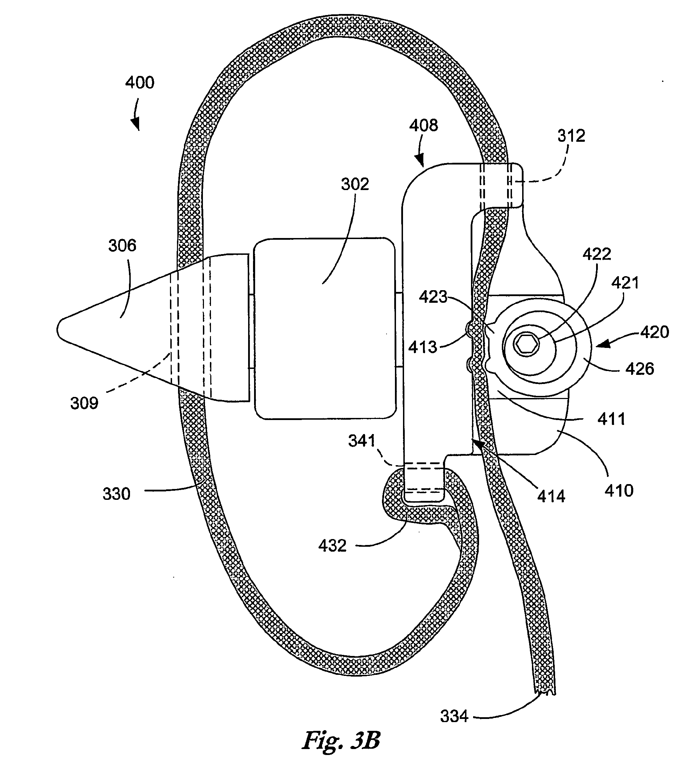

The capture device **320** and brace **308** can have alternative designs to that shown in **FIG. 3A****.** A side view of an implant **400** in accordance with an alternative embodiment of the present invention is shown in **FIG. 3B****,** the implant **400** including a capture device **420** comprising a cam **421** positioned within a ring **426** having one or more lobes **423** corresponding with one or more recesses **413** in a wall **414** of the brace **408.** The binder **330** is positioned between the capture device **420** and the brace **408.** Once the binder **330** is positioned as desired, the fastener **422** and cam **421** can be rotated using an appropriate tool, with the cam **421** forcing the lobes **423** of the ring **426** to mate with the recesses **413** of the brace **408,** preventing the ring **426** from shifting in position and defining a secure end **336** of the binder **330.** Rotating the fastener **422** rotates and optionally tightens down the cam **421.** Such a capture device **420** can provide a physician a visual indication that the binder **330** is properly secured to the brace **408,** as well as preventing slippage.

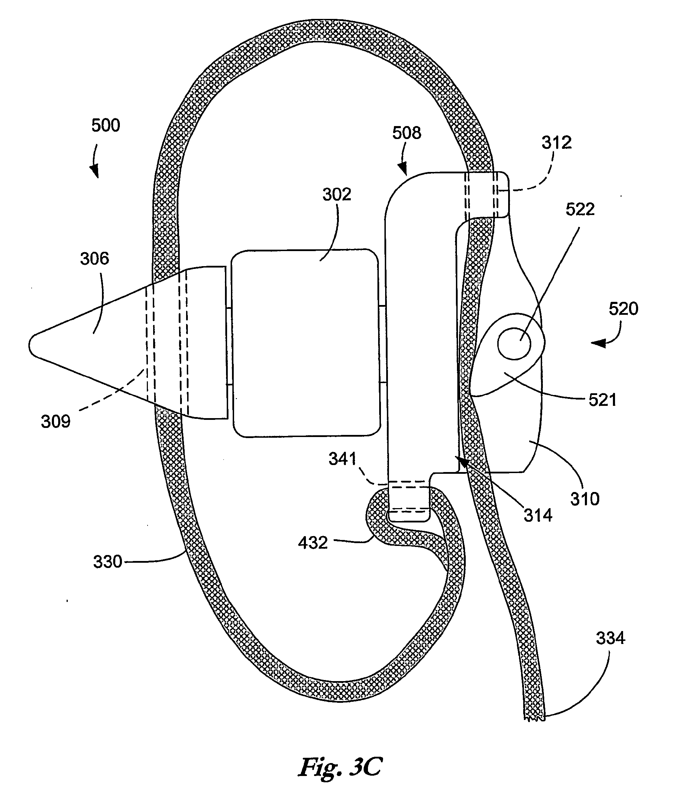

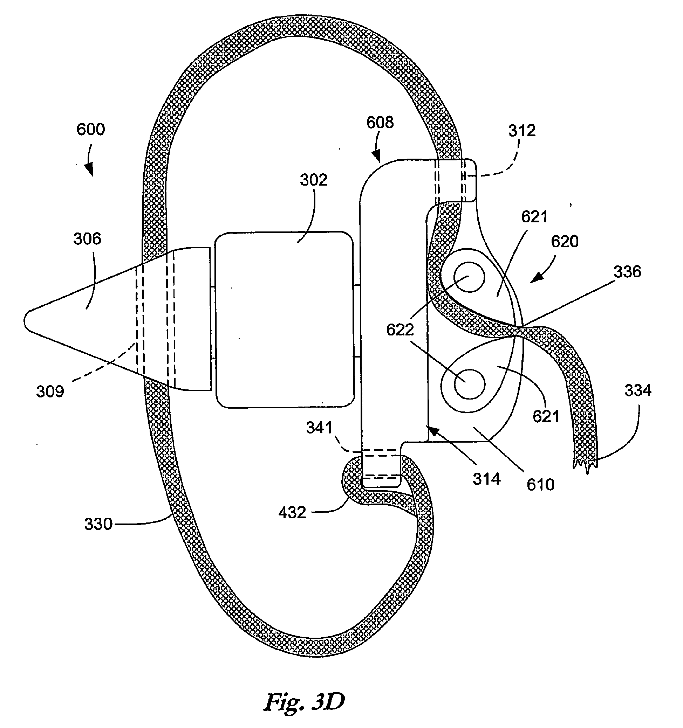

Referring to **FIGs. 3C** and **3D****,** in still other embodiments, the implant can include a capture device comprising a spring-loaded mechanism. **FIG. 3C** illustrates an implant **500** including a capture device **520** comprising a single spring-loaded cam **521** pivotally connected with the flange **310** and biased to rotate in one direction. The distance between the pivot point of the cam **510** and the wall **314** is sufficiently narrow that the rotation of the cam **521** in the direction of bias is blocked (or nearly blocked) by the wall **314.** The eccentricity of the cam **521** is large enough that a maximum gap between the wall **314** and the cam **521** is sufficiently wide as to allow the binder **330** to be threaded between the cam **521** and the wall **314.** A physician can position the binder **330** between the cam **521** and the wall **514** by overcoming the spring-force of the spring-loaded cam **521.** Once the binder **330** is position as desired, the physician need only allow the bias force of the spring-loaded cam **520** to force the cam **521** against the wall **314,** so that the cam **521** pinches and secures the binder **330** between the cam **521** and the wall **314.** Optionally, one or both of the cam **521** and the wall **314** can be knurled or otherwise textured to limit or prevent slippage. Further, the wall **314** can optionally include a recess (not shown) to receive the cam **521** so that the binder **330** is pinched within the recess (similar to the lobe and recess arrangement of **FIG. 3B**), thereby further limiting slippage.

**FIG. 3D** illustrates an implant **600** including a capture device **620** comprising dual spring-loaded cams **621,** the dual spring-loaded cams **621** being pivotally connected with the flange **310.** The dual spring-loaded cams **621** are biased in opposition to one another so that the cams **621** abut one another, similar to cam cleats commonly used for securing rope lines on boats. During surgery, the binder **330** can be loosely positioned around the adjacent spinous processes and threaded between the cams **621.** Tension can be applied to the binder **330,** as desired, by drawing the binder **330** through the cams **621.** The force of the binder **330** being pulled through the cams **621** can overcome the bias force to allow the binder **330** to be tightened, while releasing the binder **330** can define a secure end **336** of the binder **330** as the cams **621** swivel together. As above, one or both of the cams **621** can be knurled or otherwise textured to limit or prevent slippage.

Embodiments of implants have been described in **FIGs. 3A-3D** with some level of specificity; however, implants in accordance with the present invention should not be construed as being limited to such embodiments. Any number of different capture devices can be employed to fix a binder to a brace by defining a secure end of the binder, and such capture devices should not be construed as being limited to capture devices including cams, as described above. The capture device need only be a device that allows a physician to fit a binder having a generic size, or estimated size, around adjacent spinous processes with a desired level of precision in tension.

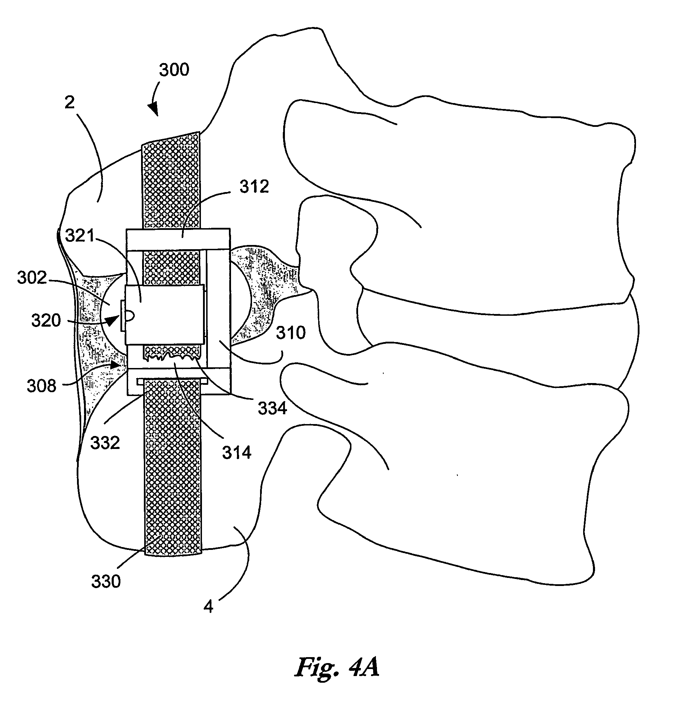

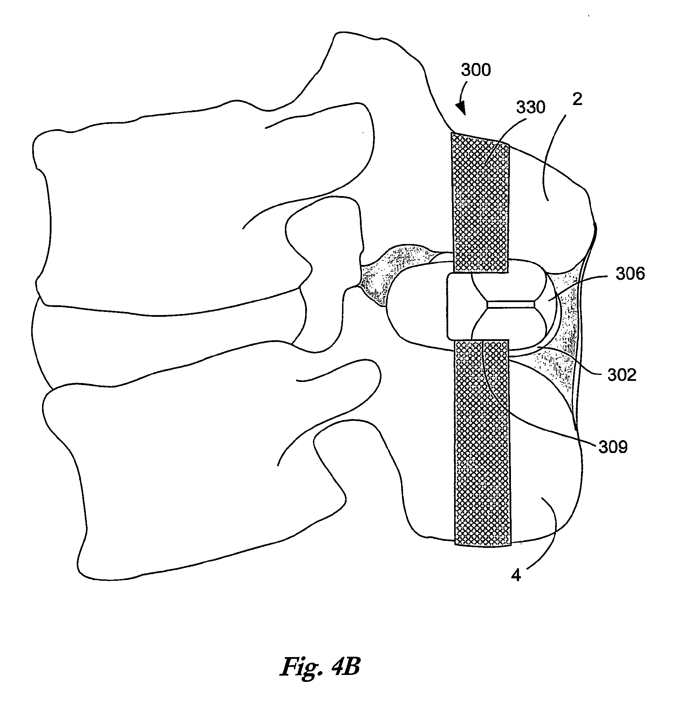

**FIG. 4A** and **4B** are an opposite end views of the implant or **FIG. 3A** positioned between adjacent spinous processes extending from vertebrae of the lumbar region. The contours of a space between adjacent spinous processes can vary between patients, and between motion segments. A rotatable spacer **302** can rotate to best accommodate the shape of the space so that the implant **300** can be positioned as desired along the spinous processes. For example, it can be desirable to position the spacer **302** as close to the vertebral bodies as possible (or as close to the vertebral bodies as practicable) to provide improved support. Once the implant **300** is positioned as desired, the binder **330** can be threaded through interspinous ligaments associated with motion segments (i.e., pairs of adjacent vertebrae and associated structures and tissues) above and below the targeted motion segment so that the binder **330** is arranged around the upper and lower spinous processes **2,4.** The binder **330** can then be threaded through the slot **312** of the brace **308** and positioned between the capture device **320** and the brace wall **314.** A first tool (not shown) can be inserted into the incision formed to insert the implant **300** between the spinous processes 2,4. Though not shown, the spacer **302** can include a notch, similar to a notch **190** of the spacer **102** of **FIG. 1****,** and the brace 308 can include recesses, similar to recesses **103,104** of the first wing **108** of **FIG. 1****,** that can be engaged by the first tool for grasping and releasing the implant **300** during insertion. (See U.S. Patent 6,712,819). Alternatively, some other technique for grasping and releasing the implant **300** can be employed. Once the implant **300** is positioned and the binder **330** is arranged as desired, a second tool (not shown), such as a forked tool having spaced apart tines, can engage the cam **321** of the capture device **320** to rotate the cam **321,** thereby securing the binder **330** to the brace **308.** A hex wrench can tighten down the fastener **322** if desired. Alternatively, a single tool can be employed to perform both the function of insertion of the implant **300** and rotation of the cam **321,** as depicted in the above referenced patent. Optionally, the binder **330** can then be trimmed so that the distal end **334** of the binder **330** does not extend undesirably away from brace **308.** As can be seen, the spacer **302** is rotated relative to the distraction guide **306** and the brace **308.** Because the spacer **302** can rotate relative to the distraction guide **306** and the brace **308,** the brace **308** can be positioned so that the binder **330** can be arranged around the upper and lower spinous processes **2,4** without twisting the binder **330.** The binder **330** is positioned around the lower spinous process **4,** threaded or positioned at least partially within a slot **309** of the distraction guide **306,** and positioned around the upper spinous process 2 so that the binder 330 can be secured to the brace **308,** as described above.

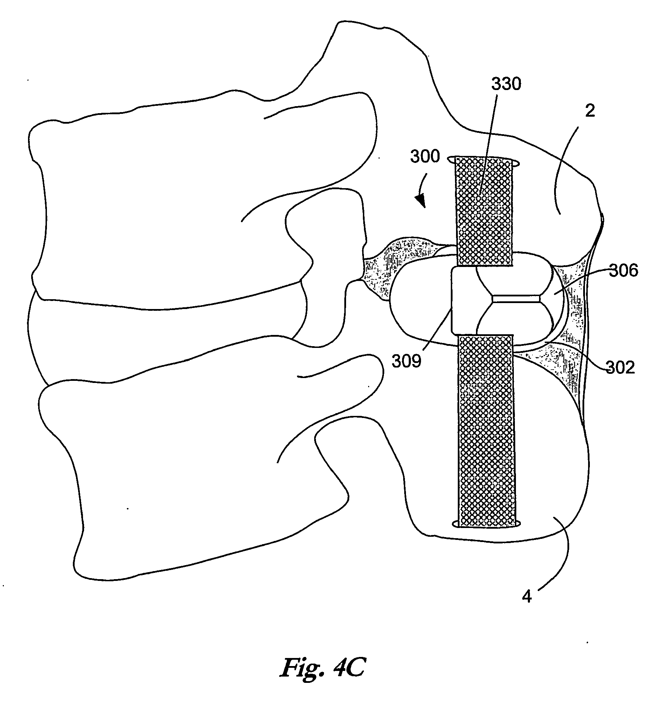

Implants in accordance with the present invention can enable a physician to limit or block flexion and extension in a targeted motion segment while minifying invasiveness of an implantation procedure (relative to implantation procedures of the prior art). However, such implants can also be used where more extensive implantation procedures are desired. For example, as shown in **FIG. 4C****,** it can be desired that the adjacent spinous processes **2,4** be surgically modified to receive the binder **330,** thereby insuring that the binder **330** does not shift or slide relative to the spinous processes **2,4.** The binder **330** is threaded directly through the respective spinous processes **2,4** rather than through the interspinous ligaments of adjacent motion segments. The amount of bone removed from the spinous processes **2,4** can be reduced where a cord or tether is used as a binder **330** rather than a strap. While such applications fall within the contemplated scope of implants and methods of implantation of the present invention, such application may not realize the full benefit that can be achieved using such implants due to the modification of the bone.

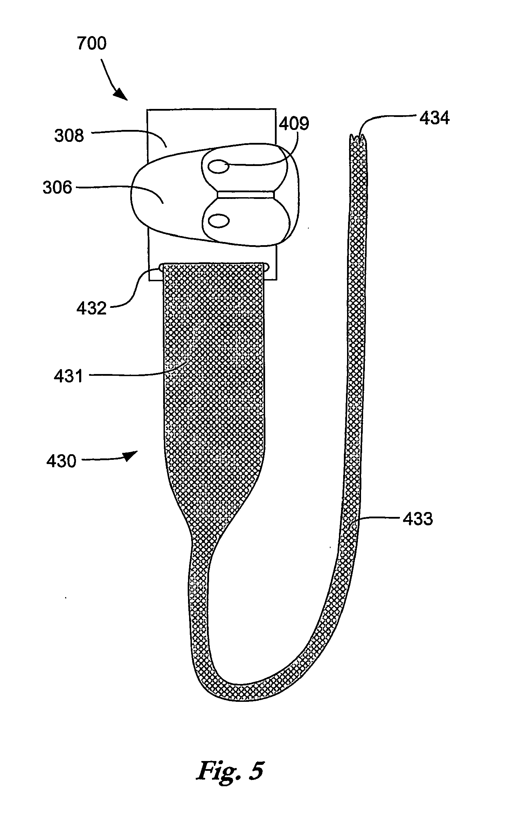

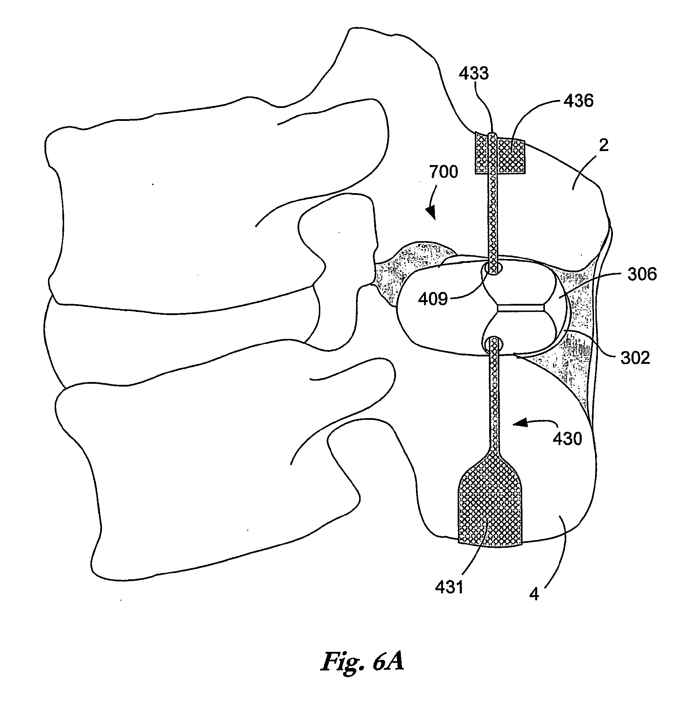

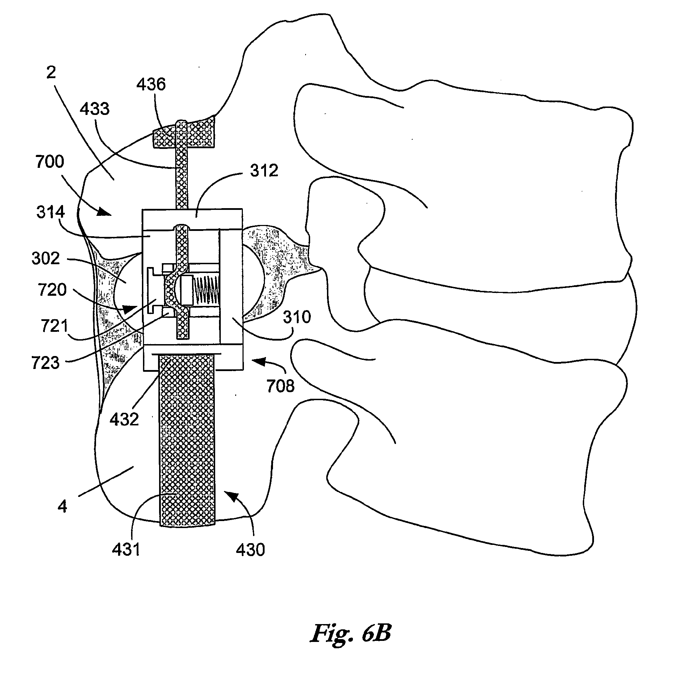

Still another embodiment of an implant **700** in accordance with the present invention is shown in the end view of **FIG. 5****.** In such an embodiment the binder **430** can comprise a first portion **431** formed as a strap for arrangement around one of the upper and lower spinous processes **2,4,** and that tapers to a second portion **433** formed as a cord. The distraction guide **406** can include a bore **409** or other cavity for receiving the second portion **433.** As can be seen in **FIG. 6A****,** once the binder **430** is threaded through the distraction guide **406,** a pad **436** of biocompatible material can be associated with the binder **430,** for example by slidably threading the binder **430** through a portion of the pad **436,** and the pad **436** can be arranged between the binder **430** and the respective spinous process **2** so that a load applied by the binder **430** is distributed across a portion of the surface of the spinous process **2.** Referring to **FIG. 6B****,** once the binder **430** is arranged as desired relative to the adjacent spinous processes **2,4,** the binder 330 can be secured by the brace **708.** The brace **708** as shown is still another embodiment of a brace for use with implants of the present invention. In such an embodiment, the brace **708** includes a capture device **720** comprising a clip including a spring-loaded button **721** having a first hole therethrough and a shell **723** in which the button **721** is disposed, the shell **723** having a second hole. A physician depresses the button **721** so that the first and second holes align. The binder **430** can then be threaded through the holes, and the button **721** can be released so that the spring forces the holes to misalign, pinching the binder **430** and defining a secure end of the binder **430.**

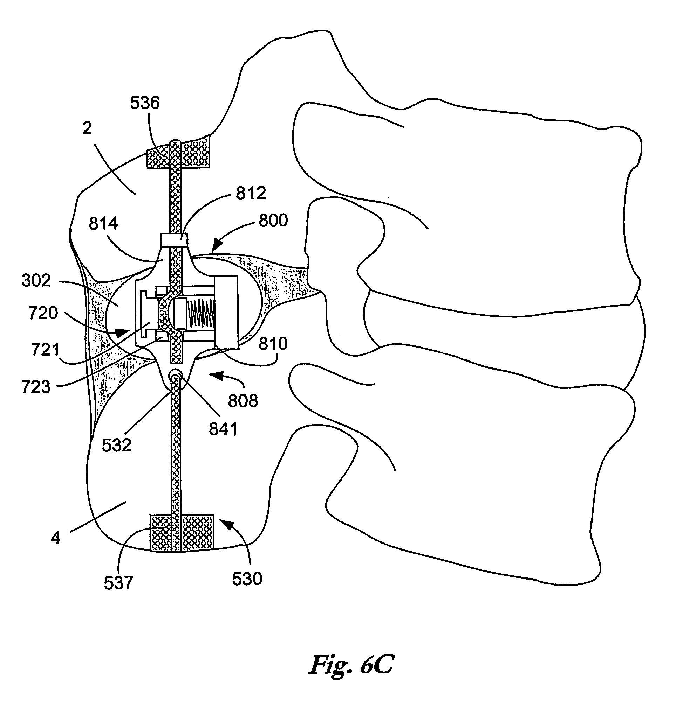

**FIG. 6C** is an end view of a still further embodiment of an implant **800** in accordance with the present invention. In such an embodiment the binder **530** can comprise a cord. An upper pad **536** and a lower pad **538** can be slidably associated with the binder **530** and arranged so that a load applied by the binder **530** is distributed across a portion of the upper and lower spinous processes **2,4.** As can be seen, such an embodiment can include a brace **808** having a substantially different shape than braces previously described. It should be noted that the brace **808** of **FIG. 6C** is shown, in part, to impress upon one of ordinary skill in the art that a brace and capture device for use with implants of the present invention can include myriad different shapes, mechanisms and arrangements, and that the present invention is meant to include all such variations. As shown, the footprint of the brace **808** is reduced by shaping the wall **814** of the brace **808** to taper at an upper end to form a guide **812** for aligning the binder **530** and to taper at a lower end to an eyelet **841** for capturing a proximal end **532** of the binder **530.** The brace **808** includes a height from eyelet **841** to guide **812** such that movement of the implant **800** in the direction of insertion is blocked or limited by the brace **808.**

Use of a binder to limit or prevent flexion can provide an additional benefit of limiting movement along the longitudinal axis **L** (shown in **FIG. 3A**). However, implants in accordance with the present invention can optionally further include a second wing for limiting or blocking movement in the direction opposite insertion. Inclusion of such a structure can ensure that the implant remains in position, for example where the binder slips out of a slot of the distraction guide, or where the binder becomes unsecured.

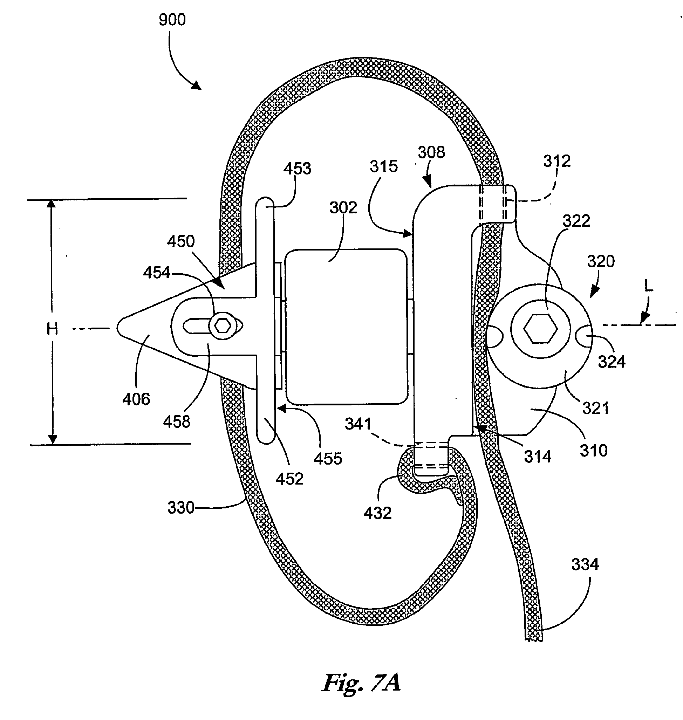

Referring to **FIG.7A****,** an implant in accordance with an embodiment can include a second wing **450** connected with the distraction guide **406** of the implant **900** by a fastener **454.** The second wing **450** is similar to the second wing 150 described above in reference to **FIG. 1****.** The second wing **450** can include an alignment tab **458** allowing a position of the second wing **450** to be adjusted along a longitudinal axis **L** of the implant **900,** and a fastener **454** (for example a hex headed bolt) for affixing the second wing **450** to the implant **900** in the position along the longitudinal axis **L** desired. The distraction guide **406** can include an alignment groove (not shown) corresponding to the alignment tab **458.** The alignment tab **458** fits within, and is movable along, the alignment groove so that a contact surface **455** of the second wing **450** can be arranged as desired. As shown, the second wing **450** includes a substantially planar contact surface arranged so that the contact surface **455** of the second wing **450** is perpendicular to the longitudinal axis **L.** However, in other embodiments, the contact surface **455** need not be planar, and can be shaped and oriented to roughly correspond with a contact surface of the upper and lower spinous processes. Likewise, a contact surface **315** of the binder **308** can be shaped and oriented to roughly correspond with a contact surface of the upper and lower spinous processes. As shown, the upper portion **453** and the lower portion **452** of the second wing **450** do not extend from the distraction guide **406** as substantially as the upper portion **153** and lower portion **152** of the second wing **150** of **FIG. 1****.** As such, the second wing **450** includes a height **H** along the spine smaller than that of the second wing **150** of **FIG.1****.** It has been observed that benefits can be gained by including a wing **450,** though the wing **450** does not extend from the distraction guide 406 as significantly as shown in **FIG.** 1 (i.e., the wing **450** includes "nubs" extending above and/or below the height of the spacer **302**). Such wings **450** will also be referred to herein as winglets. Including a second wing **450** having an overall height along the spine smaller than that of **FIG. 1** can limit movement along the longitudinal axis without interfering with (or being interfered by) the arrangement of the binder **330.**

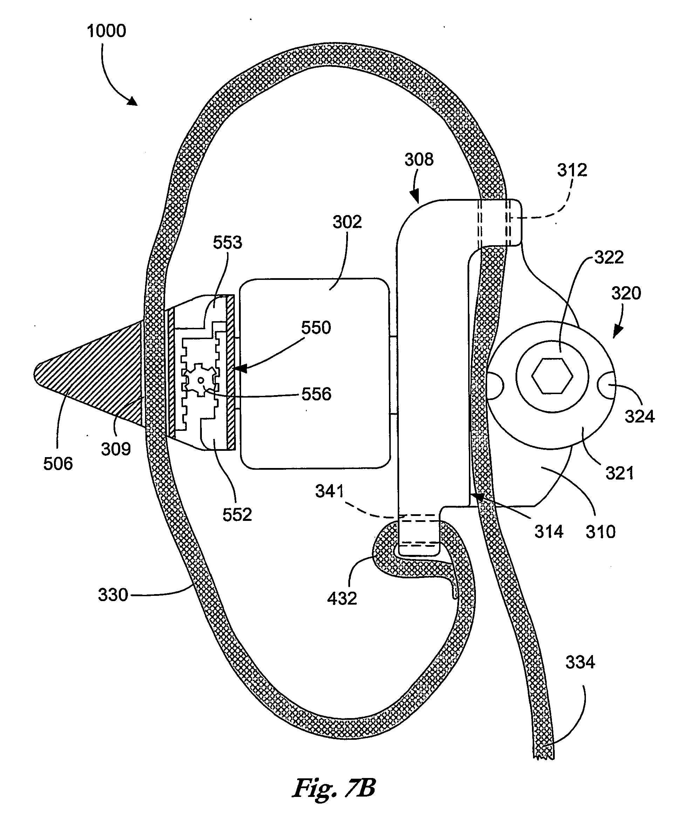

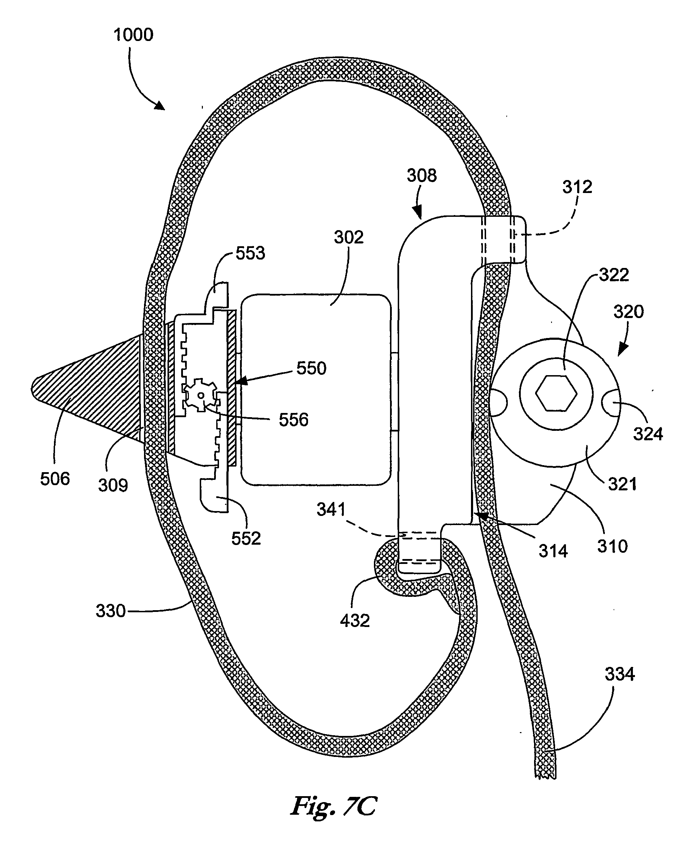

In other embodiments, implants in accordance with the present invention can include a second wing (or an upper portion and/or lower portion) extendable from the distraction guide. In this way an implant and a device for limiting or blocking movement along a longitudinal axis of the implant can be included in a single piece, possibly simplifying implantation. Referring to **FIGs. 7B** and 7C, implants **1000** in accordance with the present invention can include a distraction guide **506** having a selectably extendable upper portion **553** and lower portion **552** disposed within a cavity of the distraction guide **506.** The upper and lower portions **553,552** can be extended by actuating a nut, knob or other mechanism operably associated with a gear **556** so that the gear **556** rotates. The teeth of the gear **556** engage teeth of the upper and lower portions **553,552,** causing the upper and lower portions **553,552** to extend sufficiently that the upper and lower portions **553,552** form winglets for preventing motion of the implant **1000** in a direction opposite insertion (shown in **FIG. 7C****).** Rotating the gear **556** in an opposite direction can retract the upper and lower portions **553,552.**

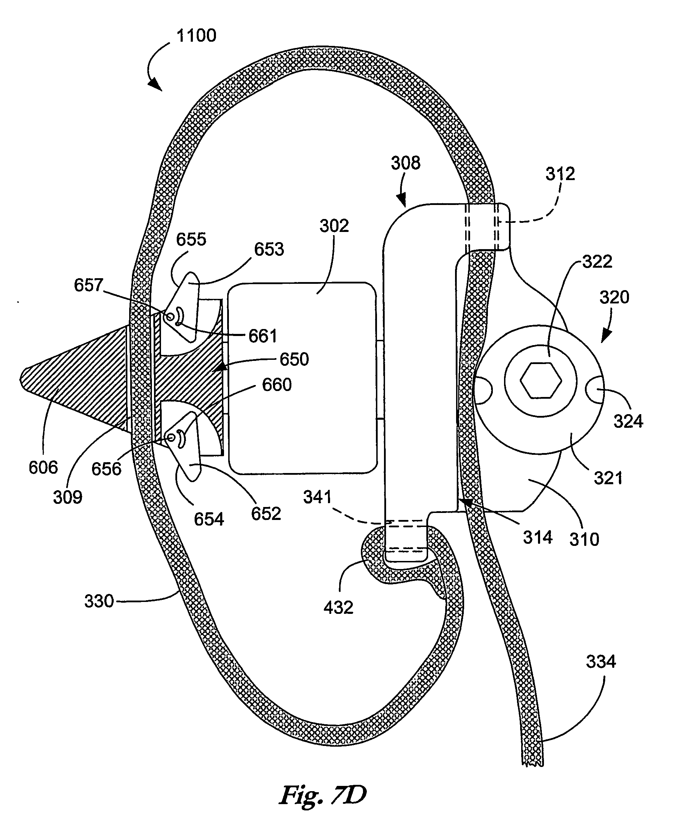

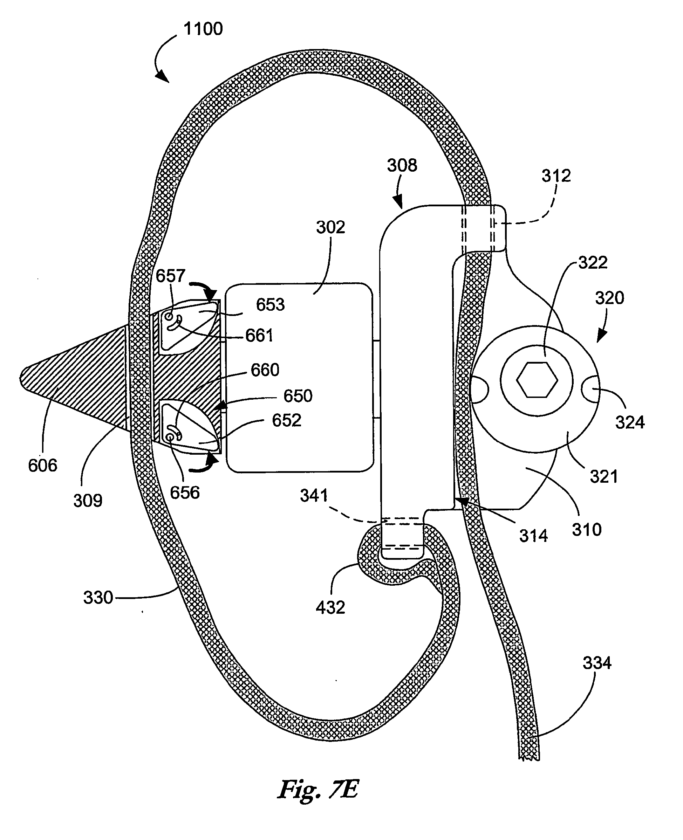

In an alternative embodiment, implants **1100** in accordance with the present invention can include spring-loaded upper and/or lower portions **653,652** such as shown in **FIGs. 7D** and **7E****.** In such an embodiment the upper and lower portions **653,652** can be fin-shaped, having sloping forward surfaces **655,654** and being spring-biased to an extended position, as shown in **FIG. 7D****.** As the implant **1100** is positioned between adj acent spinous processes, the spinous processes and/or related tissues can contact the forward surface **655,654** of the upper and lower portions **653,652,** causing the upper and lower portions **653,652** to pivot about respective hinge points **657,656** and collapse into cavities disposed within the distraction guide **606,** as shown in **FIG. 7E****.** Once the implant **1100** clears the obstruction, the upper and lower portions **653,652** re-extend out of the distraction guide **650.** A slot and pin mechanism **660,661** or other mechanism can lock the upper and lower portion **653,652** in place once extended, disallowing over-extension of the upper and lower portion **653,652** in the direction of bias. The extended upper and lower portions **653,652** limit or block movement of the implant **1100** in an a direction opposite insertion.

In still further embodiments, implants in accordance with the present invention can optionally employ some other additional mechanism for limiting or blocking motion along the longitudinal axis of the implant. Mechanisms shown and described in **FIGs. 7A-7E** are merely provided as examples of possible mechanisms for use with such implants, and are not intended to be limiting.

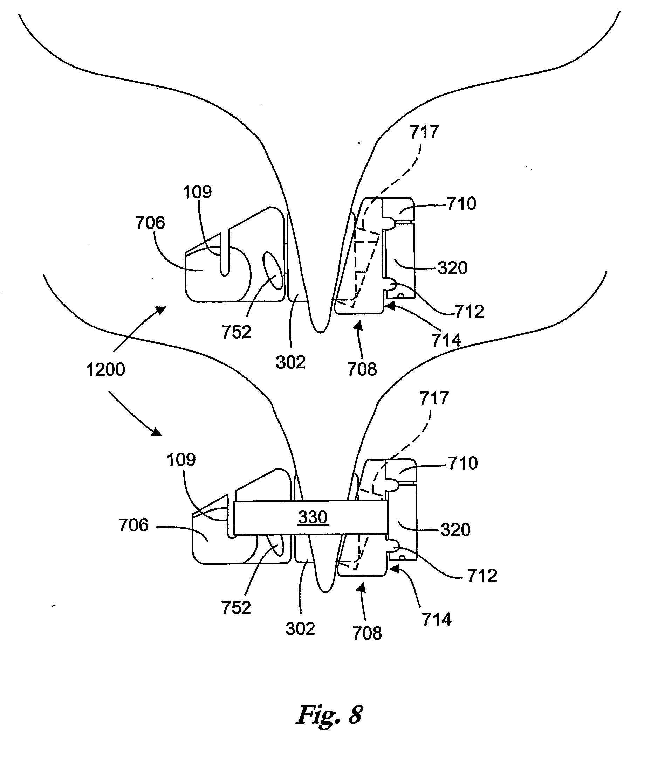

**FIG. 8** is a top-down view of still another embodiment of an implant in accordance with the present invention including a brace **708** arranged at an angle along the spinous process relative to the longitudinal axis **L** of the implant **1200.** The brace **708** is arranged at such an angle to roughly correspond to a general shape of the adjacent spinous processes. Such a general shape can commonly be found in spinous processes extending from vertebrae of the cervical and thoracic region, for example. The implant **1200** further includes a second wing **752** extending from distraction guide **706** at an angle roughly corresponding to a general shape of the adjacent spinous processes. Identical implants **1200,** one above the other, are shown. The lower implant **1200** includes a binder **330** arranged around the adjacent spinous processes (only the upper spinous process is shown) and positioned in a slot **309** of the distraction guide **706.** The binder **330** includes a capture device **320** for securing the binder **330** to the brace **708,** and a channel formed by guides **712** on the brace **708** for aligning the binder **330** with the capture device **320.** Unlike previously illustrated embodiments, the brace wall includes a recess **717** to accommodate rotation of the rotatable spacer **302.** Alternatively, the implants can include fixed spacers, for example integrally formed with the brace **708** and the distraction guide **706.**

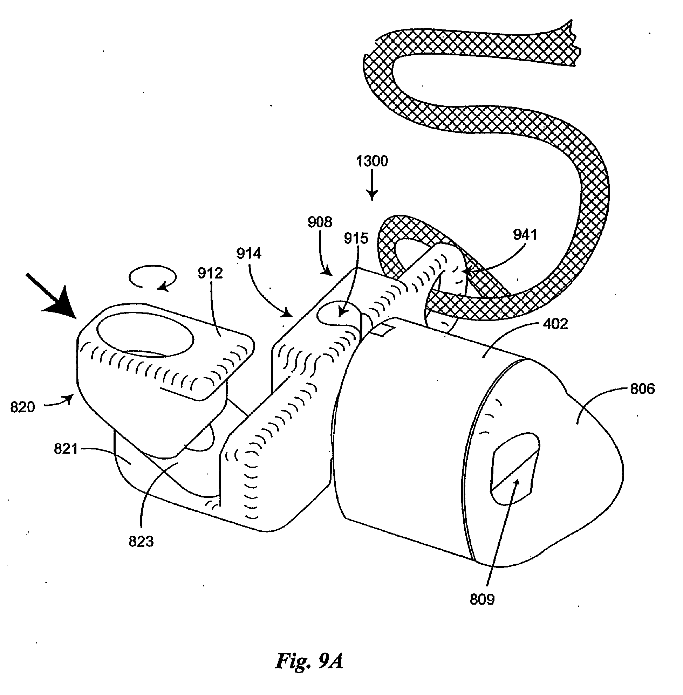

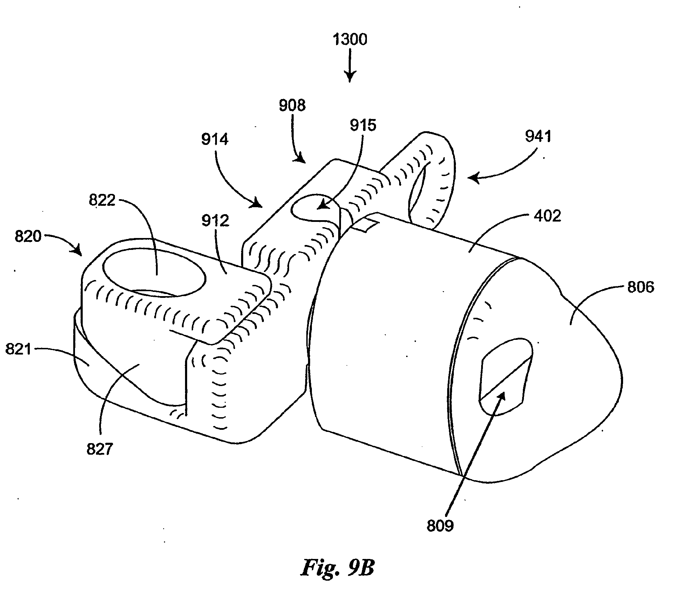

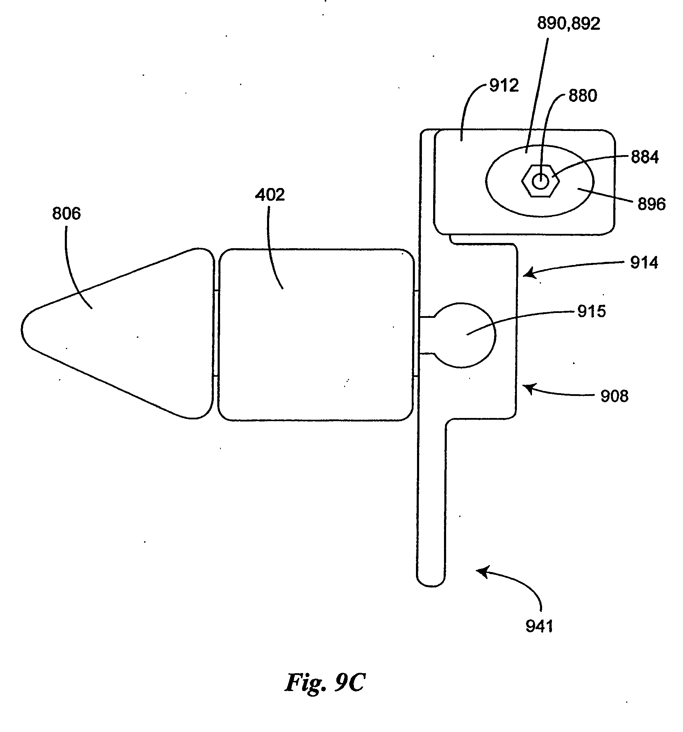

**FIGs. 9A** and **9B** are perspective views, and **FIG. 9C** is a side view of a still further embodiment of an implant in accordance with the present invention. The implant **1300** includes a distraction guide **806,** a rotatable spacer **302,** and a brace **908.** As above, a binder **330** can be fixedly connected with the brace **908** at a proximal end **332** of the binder **330.** Once positioned around adj acent spinous processes, tension of the binder **330** can be set when the binder **330** is secured to the brace **908** so that relative movement of the adjacent spinous processes during flexion is limited or prevented, as desired.

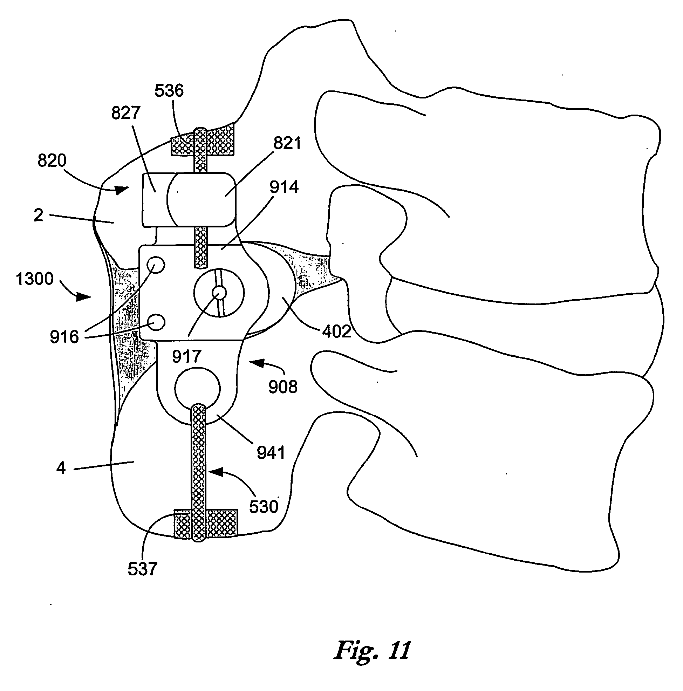

As can be seen in **FIG. 9A****,** the brace **908** can include a first end having an eyelet **941** through which the proximal end **332** of the binder **330** can be threaded and subsequently sutured, knotted or otherwise bound, or alternatively looped through the eyelet **941** and secured to itself (e.g., using a clasp) so that the proximal end **332** of the binder **330** cannot be drawn through the eyelet **941.** One of ordinary skill in the art can appreciate the myriad different ways in which the proximal end **332** of the binder **330** can be associated with the brace **908** so that tension can be applied to the binder **330.** As in previous embodiments, a free end of the binder **330** can be secured to the brace 908 by a capture device **820** associated with the brace **908.** The capture device **820** of **FIGs. 9A-11** is arranged at a second end of the brace **908** opposite the eyelet **941,** rather than approximately centered along the brace wall **914.** The brace **908** can optionally include a locking pin hole **915** that can be engaged by a locking pin of an insertion instrument (not shown), for example as described in U.S. Pat. No. 6,712,819 to Zucherman, et al.. Further, similar to implants described in Zucherman '819, the brace wall **914** can optionally include one or more holes **916** (shown in **FIG.11**) adapted to receive alignment pins of such an insertion instrument, and the spacer **402** can include a spacer engagement hole **403** adapted to receive a spacer engagement pin of such an insertion instrument. When a spacer engagement pin engages the spacer engagement hole **403,** rotation of the spacer **402** can be limited or blocked. Once the spacer engagement pin is released from the spacer engagement hole **403,** the spacer **402** can rotate and/or swivel about a central body **917** without impedance from the spacer engagement pin. Such an arrangement can provide a physician additional control over the positioning of the implant **1300,** although in other embodiments the spacer **402** need not include an engagement hole **403.** Arranging the captured device **820** at a second end of the brace **908** can allow an insertion instrument, having a configuration as described in Zucherman '819 or having some other configuration, to releasably engage the implant **1300** to assist in implantation without interference from the capture device **820.**

The distraction guide **806** of the implant **1300** can be wedge-shaped, as described above, or approximately conical, as shown in **FIGs. 9A-9C****,** and can include a slot **809** disposed through the distraction guide **806** and adapted to receive the binder **330** during implantation. Also as described above, the rotatable spacer **402** can be elliptical in cross-section, or otherwise shaped, and can rotate relative to the distraction guide **806** to roughly conform with a contour of a space between the targeted adjacent spinous processes.

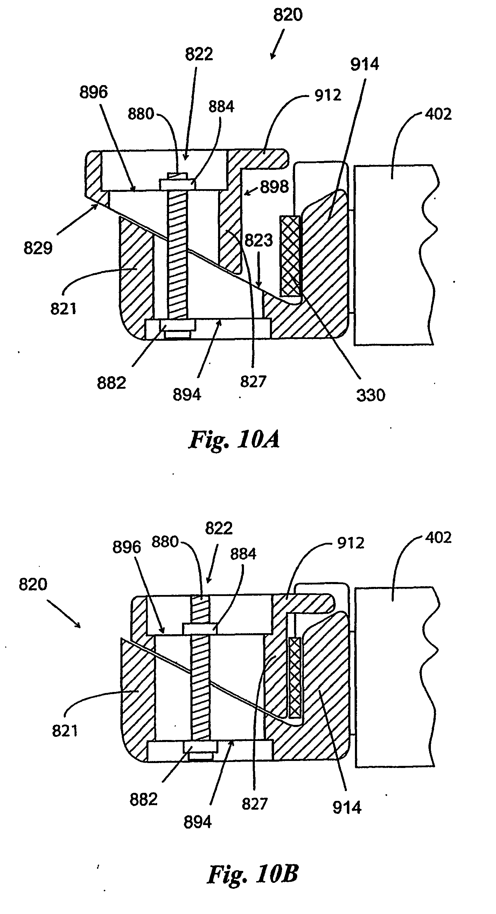

The capture device **820** is shown in cross-section in **FIGs. 10A** and **10B****.** The capture device **820** can comprise, for example, two pieces slidably associated with one another by an adjustable fastener **822** (as shown, the adjustable fastener is a hex screw). A fixed piece **821** of the capture device can extend from the brace wall **914.** The fixed piece **821** can include a beveled surface **823** that can function as a ramp. A slidable piece **827** of the capture device can be slidably associated with the fixed piece **821,** and can likewise included a beveled surface **829** positioned in opposition to the beveled surface **823** of the fixed piece **821.** As shown, the slidable piece **827** is associated with the fixed piece **821** via an adjustable fastener **822.** The fastener **822** can be positioned within slots **890,892** of the fixed piece **821** and the slidable piece **827** and can include a threaded shaft **880,** a head **882,** and a nut **884.** The head **882** of the fastener **822** can engage an anterior surface **894** of the fixed piece **821** and the nut **884** can be threaded onto the threaded shaft **880** so that the nut **884** can engage a posterior surface **896** of the slidable piece **827.** The slidable piece **827** is free to slide along the beveled surface **823** of the fixed piece **821** until both the nut **884** engages the posterior surface **896** and the head **882** engages the anterior surface **894,** blocking further movement in one direction. The distance between the anterior surface **894** and the posterior surface **896** increases or decreases as the slidable piece **827** slides along the beveled surface **823** and a distance between a capture surface **898** of the slidable piece **827** and the brace wall **914** likewise increases or decreases. The maximum distance the slidable piece **827** can travel can be defined by the distance between the nut **884** and the head **882.** A physician can adjust the maximum distance by rotating the nut **884** so that the nut **884** travels closer to, or farther from the head **882** along the threaded shaft **880,** possibly urging the capture surface **898** toward the brace wall **914.** Thus, when the implant **1300** is positioned between spinous processes, the physician can set the maximum distance so that the free end of the binder **330** can be threaded between the capture surface **898** and the brace wall **914.** As shown in **FIG. 10B****,** the physician can then adjust the fastener **822** so that the posterior surface **896** and the anterior surface **894** are urged together, the maximum distance decreases and the distance between the capture surface **898** and the brace wall **914** decreases, thereby pinching the binder **330** between the capture surface **898** and the brace wall **914** and defining a secure end of the binder **330.** In some embodiments, one or both of the capture surface **898** and the brace wall **914** can include texture so that the binder **330** is further prevented from sliding when the binder **330** is placed under increasing tension (e.g., during flexion).

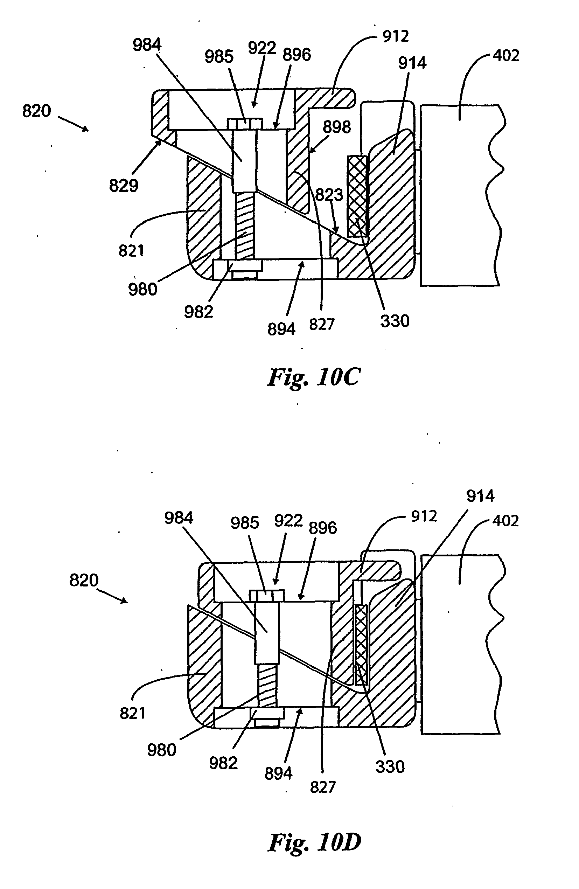

The slidable piece **827** can optionally further include a guide **912** extending from the slidable piece **827** so that the guide **912** overlaps a portion of the brace **908.** The guide **912** can extend, for example, a distance roughly similar to the maximum distance between the capture surface **898** and the brace wall **914,** and can help ensure that the binder **330** is captured between the capture surface **898** and the brace wall **914.** In other embodiments, the capture device **820** of **FIGs. 9A-10B** can include some other shape or configuration and still fall within the contemplated scope of the invention. For example, the fastener need not include a nut. In one embodiment, shown in **FIGs. 10C** and **10D****,** the fastener **922** can include a threaded shaft **980** associated with a sleeve **984.** As one of the threaded shaft **980** and the sleeve **984** is rotated, the distance between a head **982** of the threaded shaft **980** and the head **985** of the sleeve **984** can decrease or increase. In still other embodiments, the fastener need not include a threaded shaft, but rather can include a smooth shaft having a retaining clip frictionally associated with the smooth shaft. One of ordinary skill in the art will appreciate the myriad different devices that can be employed to selectively close a gap between a capture surface **898** and the brace wall **914.**

**FIG. 11** is an end view of the implant **1300** of **FIGs. 9A-10D** positioned between adjacent spinous processes. As shown, the binder 530 is a cord, but in other embodiments can have some other geometry. As described above in reference to previous embodiments, where a cord, a tether, or the like is used as a binder, a pad **536** can be arranged along a contact surface of the respective spinous process so that a load applied to the contact surface by the tension in the binder **530** can be distributed across a portion of the contact surface wider than the binder **530,** thereby reducing stress on the portion. The capture device **820** is arranged so that the slidable piece **827** is posteriorly located relative to the fixed piece **821.** A fastener **822** can be accessed by the physician using a substantially posterior approach.

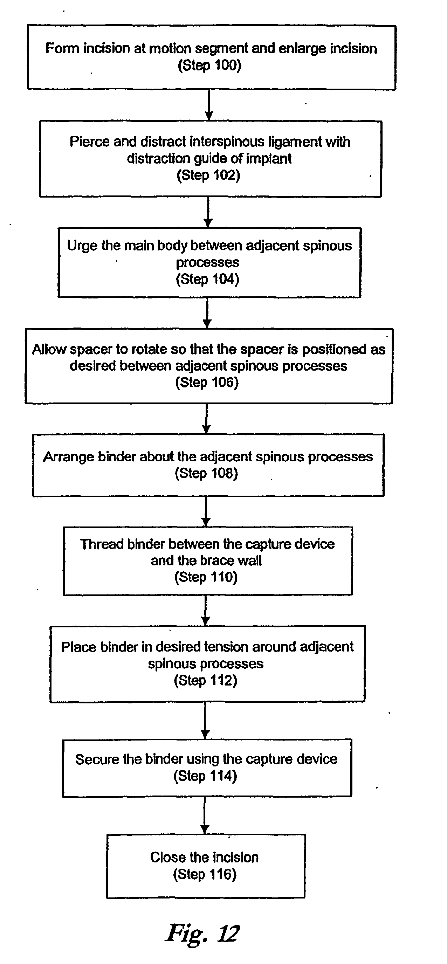

A method of surgically implanting an implant **1300** in accordance with an embodiment as described above in **FIGs. 9A-11** of the present invention is shown as a block diagram in **FIG. 12****.** The method can include forming an incision at the target motion segment, and enlarging the incision to access the target motion segment (Step 100). The interspinous ligament between targeted adjacent spinous processes can then be distracted by piercing or displacing the interspinous ligament with the distraction guide **106** (Step 102) and urging the implant **1300** between the adjacent spinous processes (Step 104). As the interspinous ligament is displaced, the spacer **302** can be positioned between the spinous processes such that the spacer **302** can rotate to assume a preferred position between the spinous processes (Step 106). Once the implant **1300** is positioned, the binder **330** can be threaded between interspinous ligaments of adjacent motion segments so that the targeted adjacent spinous processes are disposed within a loop formed by the binder **330** (Step 108). The physician can then thread the binder **330** between the capture surface **898** of the capture device **820** and the brace wall **914** (Step 110). Once a desired tension of the binder **330** is applied (Step 112), the physician can adjust the fastener **822** of the capture device **820** so that the binder **330** is secured between the captured surface **898** and the brace wall **914** (Step 114). The incision can subsequently be closed (Step 116).

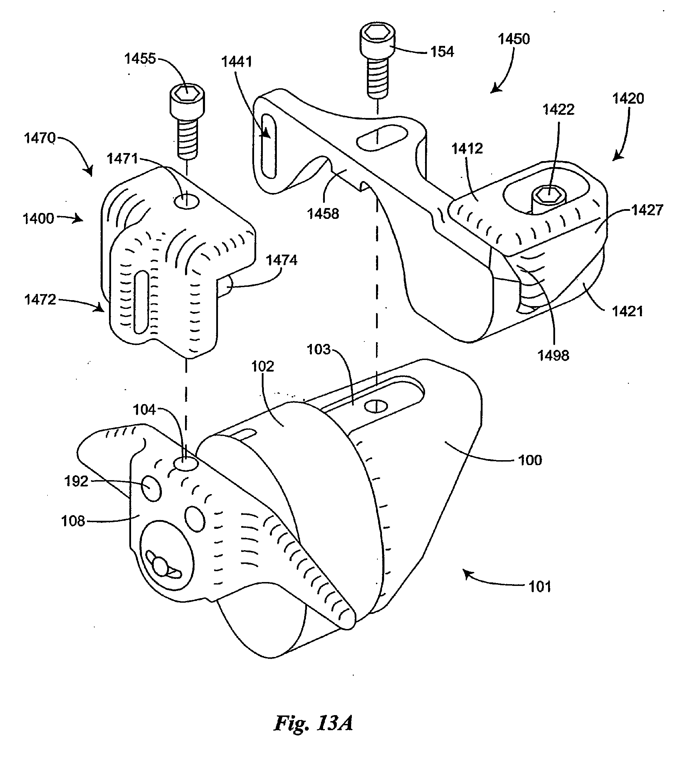

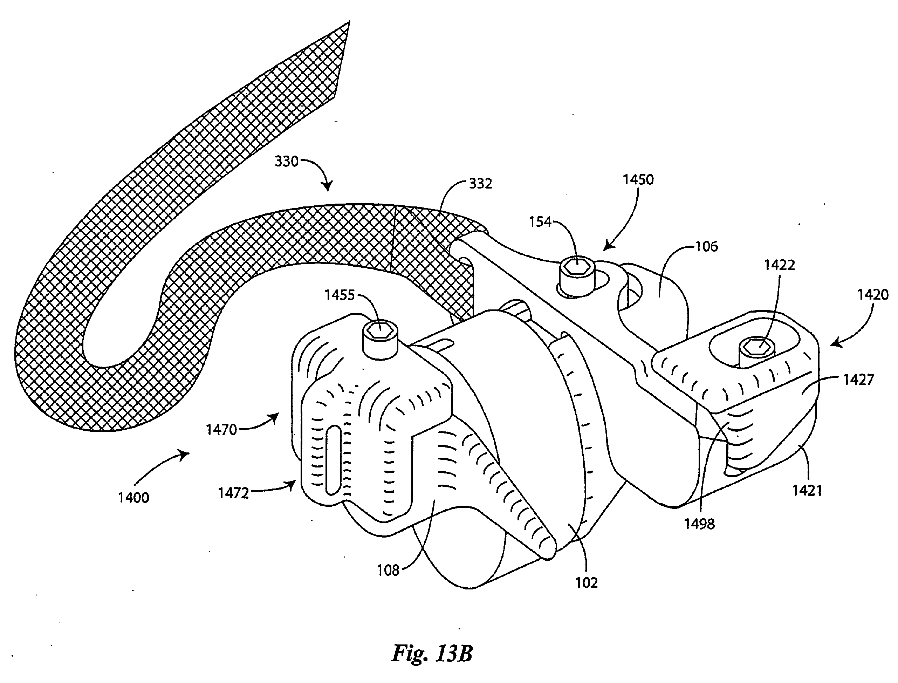

**FIG. 13A** and **13B** are perspective views of still another embodiment of an implant **1400** in accordance with the present invention. In such an embodiment, the implant **1400** can include a main body **101** similar to the main body **101** described above in reference to **FIG.1****.** As above, the main body **101** (also referred to herein as a first unit) includes a spacer **102,** a first wing **108,** a distraction guide **106** and an alignment track **103.** The main body **101** is inserted between adjacent spinous processes. Preferably, the main body **101** remains (where desired) in place without attachment to the bone or ligaments.

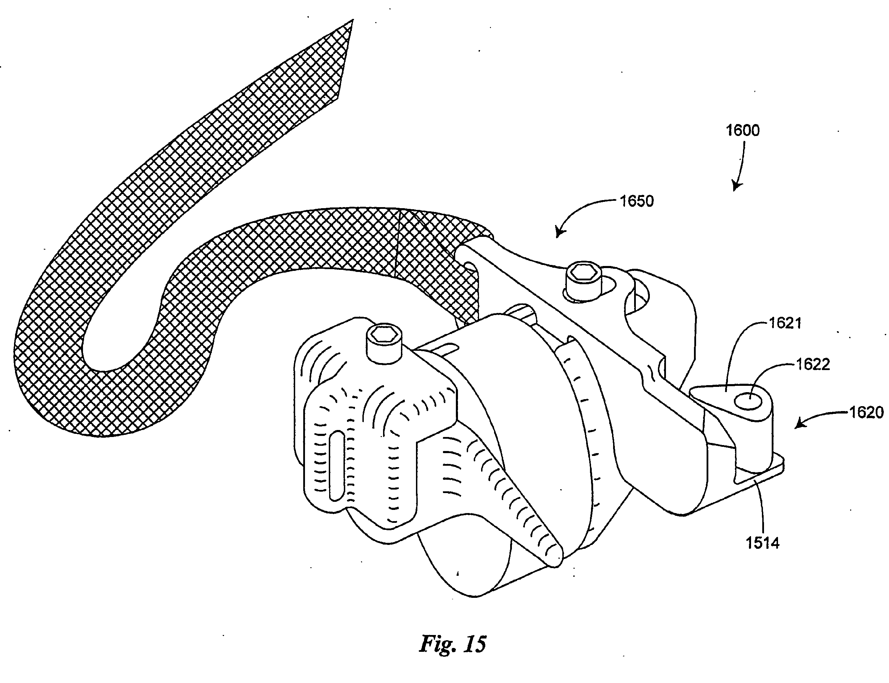

The alignment track **103** includes a threaded hole for receiving a fastener. The alignment track **103** need not include a threaded hole, but rather alternatively can include some other mechanism for fixedly connecting an additional piece (such as a second wing for limiting or blocking movement of an implant along the longitudinal axis). For example, in an alternative embodiment, the alignment track **1403** can include a flange so that the second wing **1450** can be slidably received, as shown in **FIG. 15****.**

As further shown in **FIGS.** 13A and **13B****,** the implant **1400** includes a second wing **1450** removably connectable with the implant **1400.** The second wing **1450** includes an alignment tab **1458** adapted to be received in the alignment track **103** of the main body **101**, the alignment tab **1458** optionally including a slot for receiving the fastener so that the alignment tab **1458** is disposed between the fastener and the alignment track **103**. In alternative embodiments, the alignment tab **1458** need not include a slot but rather can include some other mechanism for mating with the main body **101.**

The second wing **1450** can include a first end having a slot (or eyelet) **1441** through which the proximal end (also referred to herein as an anchored end) **332** of a binder **330** can be threaded and subsequently sutured, knotted or otherwise bound, or alternatively looped through the slot **1441** and secured to itself (e.g., using a clasp) so that the proximal end **332** of the binder **330** cannot be withdrawn through the slot **1441.** One of ordinary skill in the art can appreciate the myriad different ways in which the proximal end **332** of the binder **330** can be associated with the second wing **1450** so that tension can be applied to the binder **330**. The binder **330** can be disposed around adjacent spinous processes and a portion of the length of the binder **330** (the length of the binder being that portion of the binder extending from the proximal end of the binder) can be secured to the second wing **1450** by a capture device **1420** associated with the second wing **1450**.

The capture device **1420** of **FIGs. 13A** and **13B** is arranged at a second end of the second wing **1450** opposite the slot **1441.** The capture device **1420** can be substantially similar to capture devices **1420** as described above in reference to **FIGs. 10A** and **10B****,** and can comprise, for example, two pieces slidably associated with one another by an adjustable fastener. As above, a fixed piece **1421** of the capture device can extend from the second wing **1450.** The fixed piece **1421** can include a beveled surface that can function as a ramp. A slidable piece **1427** of the capture device can be slidably associated with the fixed piece **1421** (for example, via the adjustable fastener) and can likewise included a beveled surface positioned in opposition to the beveled surface of the fixed piece **1421.** As the slidable piece **1427** slides along the beveled surface of the fixed piece **1421,** a distance between a capture surface **1498** of the slidable piece **1427** and the second wing **1450** increases or decreases. As above, the slidable **1427** can optionally further include a guide **1412** extending from the slidable piece **1427** so that the guide **1412** overlaps a portion of the second wing **1450.** The guide **1412** can extend, for example, a distance roughly similar to the maximum distance between the capture surface **1498** and the second wing **1450,** and can help ensure that the binder **330** is arranged between the capture surface **1498** and the second wing **1450.**

A physician can position the binder **330** so that the binder **330** is disposed between adjacent spinous processes, threading the binder **330** between the slidable piece **1427** and the second wing **1450.** The physician can then adjust the fastener **1422** so that the distance between the capture surface **1498** and the second wing **1450** decreases, thereby pinching the binder **330** between the capture surface **1498** and the second wing **1450** and defining a secure end of the binder **330.** In some embodiments, one or both of the capture surface **1498** and the second wing **1450** can include texture so that the binder **330** is further prevented from sliding when the binder **330** is placed under increasing tension (e.g., during flexion).

The implant **1400** can further include a binder aligner **1470** selectably connectable with the first wing **108** of the main body **101.** The binder aligner **1470** can be connected with the first wing **108** by fastening the binder aligner **1470** to the locking pin hole **104** of the first wing **108.** In such embodiments where a fastener **1455** is used to connect the binder aligner **1470** with the first wing **108** through a hole **1471** in the binder aligner **1470,** it is desirable that the locking pin hole **104** be threaded, or otherwise adapted to receive the fastener **1455.** The locking pin hole **104** can thus be adapted to function as a hole to slidably (and temporarily) receive a locking pin of an insertion tool (not shown), thereby facilitating insertion and positioning of the main body **101,** and can also be adapted to function to fixedly receive a fastener **1455** for positioning the binder aligner **1470.** The binder aligner **1470** can optionally include pins **1474** corresponding to the alignment holes **192** of the main body **101** to further secure the binder aligner **1470** to the main body **101** and limit undesired movement of the binder aligner **1470** relative to the main body **101.**

The binder aligner **1470** includes a guide **1472** extending from the binder aligner **1470** to limit or block shifting of the binder **330** in a posterior-anterior direction. The guide **1472** can include a loop, as shown in **FIG. 13A****,** or alternatively some other structure, closed or unclosed, for limiting or blocking shifting of the binder **330.** Such a structure can prevent undesired relative movement between the binder **330** and the main body **101,** and can additionally ease arrangement of the binder **330** during an implantation procedure, by helping to aid proper positioning of the binder **330.**

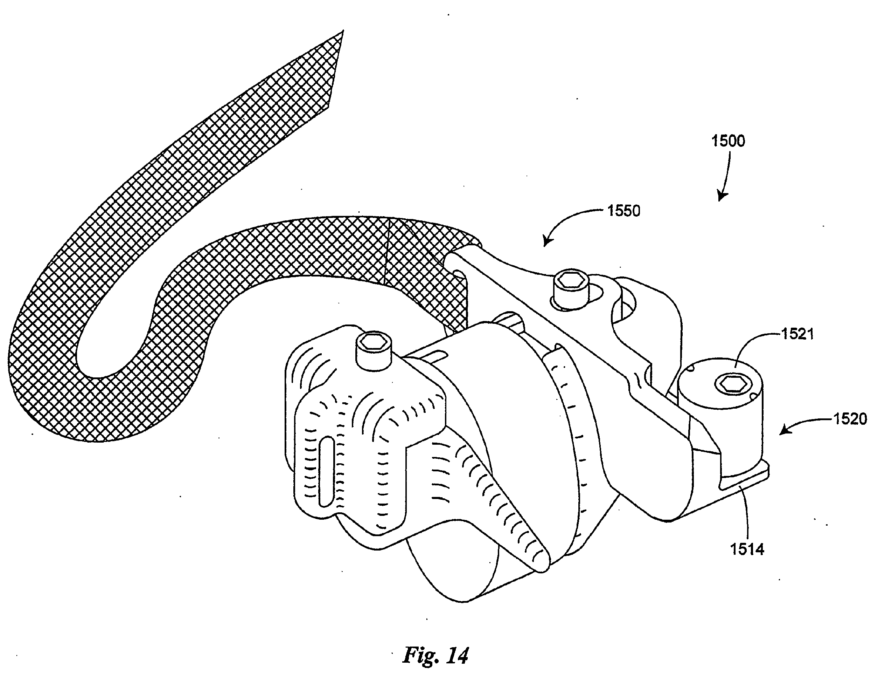

In other embodiments, the capture device of **FIGs. 13A** and **13B** can include some other shape, configuration, and mechanism and still fall within the contemplated scope of the invention. For example, referring to **FIG. 14****,** in other embodiments, a flange **1514** can extend from the second wing **1550,** from which a rotatable cam **1521** extends so that the binder **330** can be captured between the second wing **1550** and the cam **1521.** Such a capture device can resemble capture devices **1520** as described above in **FIGs. 3C** and **3B****.** Referring to **FIG. 15****,** in still other embodiments, a spring-loaded cam **1621** extends from the flange **1514** so that the binder **330** can be captured between the second wing **1514** and the spring-loaded cam **1621.** Such a capture device can resemble capture devices **1520** as described above in **FIGs. 3C** and **3D****.** In still further embodiments in accordance with the present invention, some other mechanism can be employed as a capture device associated with the second wing **1550** for securing the length of the binder **330,** for example as otherwise described in herein, and other obvious variations. One of ordinary skill in the art will appreciate the myriad different mechanisms for securing the binder **330** to the second wing **1450.**

A system in accordance with the present invention can comprise a second wing **1450** including a capture device **1420** as described above and optionally a binder aligner **1470.** The system can be used with a main body **101** in substitution for a second wing **150** as described above in **FIG.1****.** Alternatively, the system can optionally be used to modify a main body **101** previously implanted in a patient, for example by removing an existing second wing **150** and replacing the second wing **150** with the system, to additionally limit flexion as well as extension. Such a system can provide flexibility to a physician by allowing the physician to configure or reconfigure an implant according to the needs of a patient. Further, such a system can reduce costs by reducing the variety of components that need be manufactured to accommodate different procedures and different treatment goals.

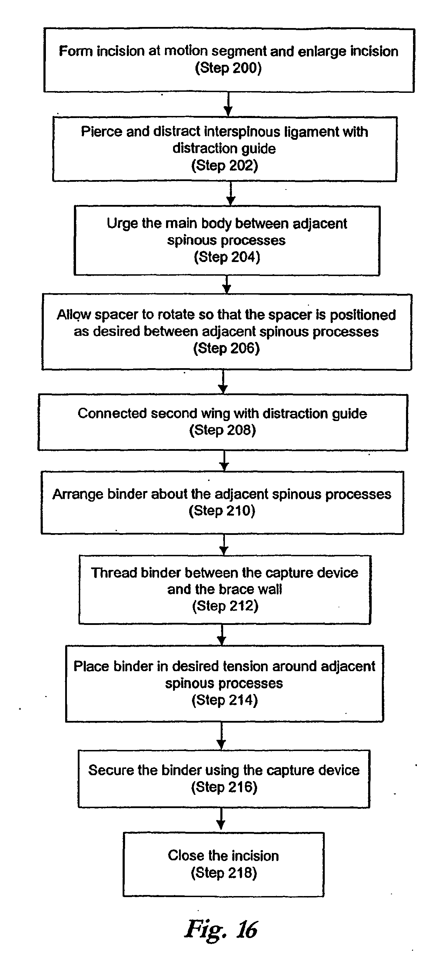

A method not claimed, of surgically implanting an implant **1400** in accordance with an embodiment as described above in **FIGs. 13A-15** of the present invention is shown as a block diagram in **FIG. 16****.** The method can include forming an incision at the target motion segment, and enlarging the incision to access the target motion segment (Step 200). The interspinous ligament between targeted adjacent spinous processes can then be distracted by piercing or displacing the interspinous ligament with the distraction guide **106** (Step 202) and urging the implant **1400** between the adjacent spinous processes (Step 204). As the interspinous ligament is displaced, the spacer **102** can be positioned between the spinous processes such that the spacer **102** can rotate to assume a preferred position between the spinous processes (Step 206). Once the implant **1400** is positioned, the second wing **1450** can be fixedly connected with the distraction guide **106** (Step 208). A binder **330** associated with the second wing **1450** can be threaded between interspinous ligaments of adjacent motion segments so that the targeted adjacent spinous processes are disposed within a loop formed by the binder **330** (Step 210). The physician can then thread the binder **330** between the capture surface **1498** of the capture device **1420** and the second wing **1450** (Step 212). Once a desired tension of the binder **330** is applied (Step 214), the physician can adjust the fastener **1422** of the capture device **1420** so that the binder **330** is secured between the captured surface **1498** and the second wing **1450** (Step 216). The incision can subsequently be closed (Step 218).

### MATERIAL FOR USE IN IMPLANTS OF THE PRESENT INVENTION

In some embodiments, the implant can be fabricated from medical grade metals such as titanium, stainless steel, cobalt chrome, and alloys thereof, or other suitable implant material having similar high strength and biocompatible properties. Additionally, the implant can be at least partially fabricated from a shape memory metal, for example Nitinol, which is a combination of titanium and nickel. Such materials are typically radiopaque, and appear during x-ray imaging, and other types of imaging. Implants in accordance with the present invention, and/or portions thereof can also be fabricated from somewhat flexible and/or deflectable material. In these embodiments, the implant and/or portions thereof can be fabricated in whole or in part from medical grade biocompatible polymers, copolymers, blends, and composites of polymers. A copolymer is a polymer derived from more than one species of monomer. A polymer composite is a heterogeneous combination of two or more materials, wherein the constituents are not miscible, and therefore exhibit an interface between one another. A polymer blend is a macroscopically homogeneous mixture of two or more different species of polymer. Many polymers, copolymers, blends, and composites of polymers are radiolucent and do not appear during x-ray or other types of imaging. Implants comprising such materials can provide a physician with a less obstructed view of the spine under imaging, than with an implant comprising radiopaque materials entirely. However, the implant need not comprise any radiolucent materials.

One group of biocompatible polymers is the polyaryletherketone group which has several members including polyetheretherketone (PEEK), and polyetherketoneketone (PEKK). PEEK is proven as a durable material for implants, and meets the criterion of biocompatibility. Medical grade PEEK is available from Victrex Corporation of Lancashire, Great Britain under the product name PEEK-OPTIMA. Medical grade PEKK is available from Oxford Performance Materials under the name OXPEKK, and also from CoorsTek under the name BioPEKK. These medical grade materials are also available as reinforced polymer resins, such reinforced resins displaying even greater material strength. In an embodiment, the implant can be fabricated from PEEK 450G, which is an unfilled PEEK approved for medical implantation available from Victrex. Other sources of this material include Gharda located in Panoli, India. PEEK 450G has the following approximate properties:

| Property | Value |
|---|---|
| Density | 1.3 g/cc |
| Rockwell M | 99 |
| Rockwell R | 126 |
| Tensile Strength | 97 MPa |
| Modulus of Elasticity | 3.5 GPa |
| Flexural Modulus | 4.1 GPa |

PEEK 450G has appropriate physical and mechanical properties and is suitable for carrying and spreading a physical load between the adjacent spinous processes. The implant and/or portions thereof can be formed by extrusion, injection, compression molding and/or machining techniques.

It should be noted that the material selected can also be filled. Fillers can be added to a polymer, copolymer, polymer blend, or polymer composite to reinforce a polymeric material. Fillers are added to modify properties such as mechanical, optical, and thermal properties. For example, carbon fibers can be added to reinforce polymers mechanically to enhance strength for certain uses, such as for load bearing devices. In some embodiments, other grades of PEEK are available and contemplated for use in implants in accordance with the present invention, such as 30% glass-filled or 30% carbon-filled grades, provided such materials are cleared for use in implantable devices by the FDA, or other regulatory body. Glass-filled PEEK reduces the expansion rate and increases the flexural modulus of PEEK relative to unfilled PEEK. The resulting product is known to be ideal for improved strength, stiffness, or stability. Carbon-filled PEEK is known to have enhanced compressive strength and stiffness, and a lower expansion rate relative to unfilled PEEK. Carbon-filled PEEK also offers wear resistance and load carrying capability.

As will be appreciated, other suitable similarly biocompatible thermoplastic or thermoplastic polycondensate materials that resist fatigue, have good memory, are flexible, and/or deflectable, have very low moisture absorption, and good wear and/or abrasion resistance, can be used without departing from, the scope of the invention. As mentioned, the implant can be comprised of polyetherketoneketone (PEKK). Other material that can be used include polyetherketone (PEK), polyetherketoneetherketoneketone (PEKEKK), polyetheretherketoneketone (PEEKK), and generally a polyaryletheretherketone. Further, other polyketones can be used as well as other thermoplastics. Reference to appropriate polymers that can be used in the implant can be made to the following documents. These documents include: PCT Publication WO 02/02158 A1, dated January 10, 2002, entitled "Bio-Compatible Polymeric Materials;" PCT Publication WO 02/00275 A1, dated January 3, 2002, entitled "Bio-Compatible Polymeric Materials;" and, PCT Publication WO 02/00270 A1, dated January 3, 2002, entitled "Bio-Compatible Polymeric Materials." Other materials such as Bionate^{®}, polycarbonate urethane, available from the Polymer Technology Group, Berkeley, California, may also be appropriate because of the good oxidative stability, biocompatibility, mechanical strength and abrasion resistance. Other thermoplastic materials and other high molecular weight polymers can be used.

As described above, the binder can be made from a biocompatible material. In an embodiment, the binder can be made from a braided polyester suture material. Braided polyester suture materials include, for example, Ethibond, Ethiflex, Mersilene, and Dacron, and are nonabsorbable, having high tensile strength, low tissue reactivity and improved handling. In other embodiments, the binder can be made from stainless steel (i.e., surgical steel), which can be braided into a tether or woven into a strap, for example, In still other embodiments, the binder can be made from some other material (or combination of materials) having similar properties.

The foregoing description of the present invention have been presented for purposes of illustration and description. It is not intended to be exhaustive or to limit the invention to the precise forms disclosed. Many modifications and variations will be apparent to practitioners. skilled in this art. The embodiments were chosen and described in order to best explain the principles of the invention and its practical application, thereby enabling others skilled in the art to understand the invention for various embodiments and with various modifications as are suited to the particular use contemplated. It is intended that the scope of the invention be defined by the following claims and their equivalents.

## Claims

1. An implant (300, 400, 500, 600, 700, 800, 900, 1000, 1100, 1200, 1300) for relieving pain associated with at least one of the spine and surrounding tissues and structures, which implant is positionable between spinous processes of the spine, the implant comprising:
a body (301), a distraction guide (306,406, 506, 606, 706, 806) extending from a distal end of said body and further including a binder aligner (309, 809) therein;
a brace (308, 408, 708, 808, 908) disposed adjacent to a proximal end of said body;
a binder (330, 430, 530) adapted to be disposed around the spinous processes, the binder having an anchor end connected with the brace and a length extending from the anchor end to a second end, the binder adapted to extend through the distraction guide; and
a capture device (320, 420, 520, 620, 720, 820) associated with the brace, wherein the capture device can secure the binder along the length.

2. The implant of claim 1, wherein:
the binder aligner is an opening defined by the distraction guide that can receive the binder so that the binder is approximately aligned along a longitudinal axis of the body.

3. The implant of claim 1, further comprising:
a spacer (302, 402) positioned over said body with said spacer being able to rotate about said longitudinal axis of said body so as to be positionable relative to said body in order to aid in positioning said implant between spinous processes.

4. The implant of claim 1, wherein:
the capture device includes a portion (321, 421, 521, 621, 721, 827) that is movably associated with the brace; and
the binder is fixedly associated with the brace.

5. The system of claim 1, further comprising;
a wing (450, 550, 650) adapted to be connected with one of the spacer and the distraction guide.

6. The implant of claim 3, wherein the spacer is substantially cylindrical in shape.

7. The implant of claim 5, wherein the wing includes a selectably extendable upper portion (553, 653) and a selectably extendable lower portion (552, 652).

## Patentansprüche

1. Implantat (300, 400, 500, 600, 700, 800, 900, 1000, 1100, 1200, 1300) zum Lindern eines Schmerzes in Verbindung mit der Wirbelsäule und/oder umgebenden Geweben und Strukturen, das zwischen Wirbelfortsätzen der Wirbelsäule positionierbar ist, mit:
einem Körper (301), wobei sich eine Distraktionsführung (306, 406, 506, 606, 706, 806) von einem distalen Ende des Körpers erstreckt und ferner ein darin vorgesehenes Bandausrichtungsmittel (309, 809) aufweist;
einer Verstrebung (308, 408, 708, 808, 908), die benachbart zu einem proximalen Ende des Körpers angeordnet ist;
einem Band (330, 430, 530), das dazu angepasst ist, um die Wirbelfortsätze angeordnet zu sein, wobei das Band ein Verankerungsende, das mit der Verstrebung verbunden ist, und einen Längenabschnitt, der sich von dem Verankerungsende zu einem zweiten Ende erstreckt, aufweist und dazu angepasst ist, sich durch die Distraktionsführung zu erstrecken; und
einer Haltevorrichtung (320, 420, 520, 620, 720, 820), die zu der Verstrebung gehört, wobei die Haltevorrichtung das Band entlang des Längenabschnitts festhalten kann.

2. Implantat nach Anspruch 1, bei dem:
das Bandausrichtungsmittel eine durch die Distraktionsführung festgelegte Öffnung ist, die das Band so aufnehmen kann, dass das Band annähernd entlang einer Längsachse des Körpers ausgerichtet ist.

3. Implantat nach Anspruch 1, ferner mit:
einem Abstandshalter (302, 402), der über dem Körper positioniert ist, wobei der Abstandshalter dazu in der Lage ist, sich um die Längsachse des Körpers zu drehen, so dass er zur Unterstützung der Positionierung des Implantats zwischen Wirbelfortsätzen relativ zu dem Körper positionierbar ist.

4. Implantat nach Anspruch 1, bei dem:
die Haltevorrichtung einen Teil (321, 421, 521, 621, 721, 827) aufweist, der bezüglich der Verstrebung bewegbar ist; und
das Band bezüglich der Verstrebung fixiert ist.

5. System nach Anspruch 1, ferner mit:
einem Flügel (450, 550, 650), der dazu angepasst ist, mit dem Abstandshalter oder der Distraktionsführung verbunden zu sein.

6. Implantat nach Anspruch 3, bei dem der Abstandshalter im Wesentlichen zylindrisch geformt ist.

7. Implantat nach Anspruch 5, bei dem der Flügel einen selektiv ausfahrbaren oberen Teil (553, 653) und einen selektiv ausfahrbaren unteren Teil (552, 652) aufweist.

## Revendications

1. Implant (300, 400, 500, 600, 700, 800, 900, 1000, 1100, 1200, 1300) pour soulager des douleurs associées avec au moins un élément parmi la colonne vertébrale et les tissus et structures environnantes, ledit implant pouvant être positionné entre des apophyses épineuses de la colonne vertébrale, l'implant comprenant :
un corps (301), un guide d'écartement (306, 406, 506, 606, 706, 806) s'étendant depuis une extrémité distale dudit corps et incluant en outre un moyen d'alignement et de liaison (309, 809) à l'intérieur ;
un tirant (308, 408, 708, 808, 908) disposé adjacent à une extrémité proximale dudit corps ;
un moyen de liaison (330, 430, 530) adapté à être disposé autour des apophyses épineuses, le moyen de liaison ayant une extrémité d'ancrage connectée au tirant et une longueur s'étendant depuis l'extrémité d'ancrage jusqu'à une seconde extrémité, le moyen de liaison étant adapté à s'étendre à travers le guide d'écartement ; et
un dispositif de capture (320, 420, 520, 620, 720, 820) associé au tirant, de sorte que le dispositif de capture peut fixer le moyen de liaison le long de la longueur.

2. Implant selon la revendication 1, dans lequel :
le moyen d'alignement et de liaison est une ouverture définie par le guide d'écartement, qui est capable de recevoir le moyen de liaison de sorte que le moyen de liaison est approximativement aligné le long d'un axe longitudinal du corps.

3. Implant selon la revendication 1, comprenant en outre :
un moyen d'espacement (302, 402) positionné au-dessus dudit corps, ledit moyen d'écartement étant capable de tourner autour dudit axe longitudinal dudit corps de manière à pouvoir être positionné par rapport audit corps afin d'aider au positionnement dudit implant entre des apophyses épineuses.

4. Implant selon la revendication 1, dans lequel :
le dispositif de capture inclut une portion (321, 421, 521, 621, 721, 827) qui est associée de façon mobile avec le tirant ; et le moyen de liaison est associé de manière fixe avec le tirant.

5. Système selon la revendication 1, comprenant en outre :
une ailette (450), 550, 650) adaptée à être connectée avec un élément parmi le moyen d'espacement et le guide d'écartement.

6. Implant selon la revendication 3, dans lequel le moyen d'espacement a une forme sensiblement cylindrique.

7. Implant selon la revendication 5, dans lequel l'ailette inclut une portion supérieure capable d'être étendue de manière sélective (553, 653) et une portion inférieure capable d'être étendue de manière sélective (552, 652).
